# EUROPEAN PATENT APPLICATION

(11) **EP 3 851 528 A1**
(43) Date of publication of application: **21.07.2021**
(21) Application number: 19860511.5
(22) Date of filing: 11.09.2019
(51) Int. Cl.: C12N 15/10, C12N 15/113, C12N 15/86, C12N 15/90, C12N 9/22, A61K 45/06, A61K 48/00, A61P 35/00

(54) **COMPOSITION FOR INDUCING DEATH OF CELLS HAVING MUTATED GENE, AND METHOD FOR INDUCING DEATH OF CELLS HAVING MODIFIED GENE BY USING COMPOSITION**

(30) Priority: 12.09.2018 KR 20180109214
(71) Applicant: Institute for Basic Science, Yuseong-gu Daejeon 34126 (KR); UNIST (Ulsan National Institute of Science and Technology), Ulju-gun, Ulsan 44919 (KR)
(72) Inventor: MYUNG, Kyungjae, Ulsan 44667 (KR); KWON, Taejoon, Ulju-gun Ulsan 44920 (KR); BAEK, In-Joon, Ulju-gun Ulsan 44922 (KR); RA, Jae Sun, Ulju-gun Ulsan 44931 (KR); SEO, Yuri, Daejeon 34664 (KR); YUN, Seongmin, Gunsan-si Jeollabuk-do 54086 (KR)
(74) Representative: Mewburn Ellis LLP
(86) International application number: PCT/KR2019/011866
(87) International publication number: WO 2020/055187

(57) **Abstract**

The present invention relates to a composition for inducing death of cells having genomic sequence variations, comprising a nuclease and a cleavaging agent, and a method of inducing death of cells having genomic sequence variations.

<110>    Institute for Basic Science
         Ulsan National Institute of Science and Technology

<120>    Composition For Inducing Apoptosis of Cells With Modified Genes

<130>    PP-B2259

<150>    KR 18/109,214
<151>    2018-09-12

<160>    163

<170>    PatentIn version 3.5

<210>    1
<211>    20
<212>    DNA
<213>    Artificial Sequence

<220>
<223>    Synthetic Sequence


<400>    1
agccctagaa ttcccttcac                                                   20


<210>    2
<211>    20
<212>    DNA
<213>    Artificial Sequence

<220>
<223>    Synthetic Sequence


<400>    2
cttctccacc aattggtgtt                                                   20


<210>    3
<211>    20
<212>    DNA
<213>    Artificial Sequence

<220>
<223>    Synthetic Sequence


<400>    3
gttttgtctc attatcacgc                                                   20


<210>    4
<211>    20
<212>    DNA
<213>    Artificial Sequence

<220>
<223>    Synthetic Sequence


<400>    4

ctgtgtttat ggtgctttgt                                                    20

<210>    5
<211>    20
<212>    DNA
<213>    Artificial Sequence

<220>
<223>    Synthetic Sequence

<400>    5
tgtaagaagg ccgaatcacg                                                    20

<210>    6
<211>    20
<212>    DNA
<213>    Artificial Sequence

<220>
<223>    Synthetic Sequence

<400>    6
tcctatacgg ctctaccagt                                                    20

<210>    7
<211>    20
<212>    DNA
<213>    Artificial Sequence

<220>
<223>    Synthetic Sequence

<400>    7
gtctaaaggt tagaattccg                                                    20

<210>    8
<211>    20
<212>    DNA
<213>    Artificial Sequence

<220>
<223>    Synthetic Sequence

<400>    8
gagactgcta tcagtcatgt                                                    20

<210>    9
<211>    20
<212>    DNA
<213>    Artificial Sequence

<220>
<223>    Synthetic Sequence

<400>    9
ttttggtcaa gagcagagga                                                    20


<210>    10
<211>    20
<212>    DNA
<213>    Artificial Sequence

<220>
<223>    Synthetic Sequence


<400>    10
tgtgtgccgt aatatgggaa                                                    20


<210>    11
<211>    20
<212>    DNA
<213>    Artificial Sequence

<220>
<223>    Synthetic Sequence


<400>    11
tgaccttctg agttccttat                                                    20


<210>    12
<211>    20
<212>    DNA
<213>    Artificial Sequence

<220>
<223>    Synthetic Sequence


<400>    12
gtttgtcata ccagtcaaag                                                    20


<210>    13
<211>    20
<212>    DNA
<213>    Artificial Sequence

<220>
<223>    Synthetic Sequence


<400>    13
acacaggacc agaaaccctg                                                    20


<210>    14
<211>    20
<212>    DNA
<213>    Artificial Sequence

<220>

<223>    Synthetic Sequence

<400>    14
actcttccag ttgttcactg                                                    20

<210>    15
<211>    20
<212>    DNA
<213>    Artificial Sequence

<220>
<223>    Synthetic Sequence

<400>    15
gtgcatttct ctgctgagtc                                                    20

<210>    16
<211>    20
<212>    DNA
<213>    Artificial Sequence

<220>
<223>    Synthetic Sequence

<400>    16
tatatatctg caggatctgc                                                    20

<210>    17
<211>    20
<212>    DNA
<213>    Artificial Sequence

<220>
<223>    Synthetic Sequence

<400>    17
tctcttgctg tagagtgcgt                                                    20

<210>    18
<211>    20
<212>    DNA
<213>    Artificial Sequence

<220>
<223>    Synthetic Sequence

<400>    18
acacctgctt caggtgtgtg                                                    20

<210>    19
<211>    20
<212>    DNA
<213>    Artificial Sequence

<220>
<223>    Synthetic Sequence


<400>    19
catttaaaag gatgccagca                                                    20


<210>    20
<211>    20
<212>    DNA
<213>    Artificial Sequence

<220>
<223>    Synthetic Sequence


<400>    20
ctgatacttc tgataccaga                                                    20


<210>    21
<211>    20
<212>    DNA
<213>    Artificial Sequence

<220>
<223>    Synthetic Sequence


<400>    21
gttcagcctg agtttggagt                                                    20


<210>    22
<211>    20
<212>    DNA
<213>    Artificial Sequence

<220>
<223>    Synthetic Sequence


<400>    22
agagatacag aagtccctgt                                                    20


<210>    23
<211>    20
<212>    DNA
<213>    Artificial Sequence

<220>
<223>    Synthetic Sequence


<400>    23
agatgtgtaa ggttgcaaca                                                    20


<210>    24
<211>    20

<212>    DNA
<213>    Artificial Sequence

<220>
<223>    Synthetic Sequence


<400>    24
gaaccacaga acctggcata                                                    20


<210>    25
<211>    20
<212>    DNA
<213>    Artificial Sequence

<220>
<223>    Synthetic Sequence


<400>    25
tgacctttca caaaggccca                                                    20


<210>    26
<211>    20
<212>    DNA
<213>    Artificial Sequence

<220>
<223>    Synthetic Sequence


<400>    26
gcctcagggg aatggagata                                                    20


<210>    27
<211>    20
<212>    DNA
<213>    Artificial Sequence

<220>
<223>    Synthetic Sequence


<400>    27
tttgctactt tgctaggttt                                                    20


<210>    28
<211>    20
<212>    DNA
<213>    Artificial Sequence

<220>
<223>    Synthetic Sequence


<400>    28
agggagctca gagtcttgtg                                                    20

<210>    29
<211>    20
<212>    DNA
<213>    Artificial Sequence

<220>
<223>    Synthetic Sequence


<400>    29
ctccttccct tcctgagttt                                                    20


<210>    30
<211>    20
<212>    DNA
<213>    Artificial Sequence

<220>
<223>    Synthetic Sequence


<400>    30
gtgtgagtga gagagaaaga                                                    20


<210>    31
<211>    20
<212>    DNA
<213>    Artificial Sequence

<220>
<223>    Synthetic Sequence


<400>    31
ttatccaatc agctatggcc                                                    20


<210>    32
<211>    20
<212>    DNA
<213>    Artificial Sequence

<220>
<223>    Synthetic Sequence


<400>    32
gcttcactgg cttcactgga                                                    20


<210>    33
<211>    20
<212>    DNA
<213>    Artificial Sequence

<220>
<223>    Synthetic Sequence


<400>    33
atttgtacag tctgcttact                                                    20

<210>    34
<211>    20
<212>    DNA
<213>    Artificial Sequence

<220>
<223>    Synthetic Sequence


<400>    34
gcatcttcaa caggtgattc                                                    20


<210>    35
<211>    20
<212>    DNA
<213>    Artificial Sequence

<220>
<223>    Synthetic Sequence


<400>    35
tttcaagcat ttcaatgcag                                                    20


<210>    36
<211>    20
<212>    DNA
<213>    Artificial Sequence

<220>
<223>    Synthetic Sequence


<400>    36
ttctctctgt gcttctttga                                                    20


<210>    37
<211>    20
<212>    DNA
<213>    Artificial Sequence

<220>
<223>    Synthetic Sequence


<400>    37
tggcccttgt ggcagtttag                                                    20


<210>    38
<211>    20
<212>    DNA
<213>    Artificial Sequence

<220>
<223>    Synthetic Sequence

<400>    38
atgggattaa tgggattgct                                                    20


<210>    39
<211>    20
<212>    DNA
<213>    Artificial Sequence

<220>
<223>    Synthetic Sequence


<400>    39
tcatacagag aaagcagggc                                                    20


<210>    40
<211>    20
<212>    DNA
<213>    Artificial Sequence

<220>
<223>    Synthetic Sequence


<400>    40
tcatctcatg tcttctcatg                                                    20


<210>    41
<211>    20
<212>    DNA
<213>    Artificial Sequence

<220>
<223>    Synthetic Sequence


<400>    41
gacagaaccc aagtaatttc                                                    20


<210>    42
<211>    20
<212>    DNA
<213>    Artificial Sequence

<220>
<223>    Synthetic Sequence


<400>    42
ttatgccatc tggtccaggc                                                    20


<210>    43
<211>    20
<212>    DNA
<213>    Artificial Sequence

<220>
<223>    Synthetic Sequence

<400>    43
ccagacatac actaggcatc                                                    20

<210>    44
<211>    20
<212>    DNA
<213>    Artificial Sequence

<220>
<223>    Synthetic Sequence

<400>    44
tcccgtgagg cattctgtac                                                    20

<210>    45
<211>    20
<212>    DNA
<213>    Artificial Sequence

<220>
<223>    Synthetic Sequence

<400>    45
attcttcgtg ctgatgtacc                                                    20

<210>    46
<211>    20
<212>    DNA
<213>    Artificial Sequence

<220>
<223>    Synthetic Sequence

<400>    46
acaatctgtc cagaggccaa                                                    20

<210>    47
<211>    20
<212>    DNA
<213>    Artificial Sequence

<220>
<223>    Synthetic Sequence

<400>    47
gtgaagggca agcaaggaca                                                    20

<210>    48
<211>    20
<212>    DNA
<213>    Artificial Sequence

<220>
<223>    Synthetic Sequence


<400>    48
tgatatggca tagcgatcat                                                    20


<210>    49
<211>    20
<212>    DNA
<213>    Artificial Sequence

<220>
<223>    Synthetic Sequence


<400>    49
taacagccat gtggtgttac                                                    20


<210>    50
<211>    20
<212>    DNA
<213>    Artificial Sequence

<220>
<223>    Synthetic Sequence


<400>    50
ggaaacagca gcagtgcaca                                                    20


<210>    51
<211>    20
<212>    DNA
<213>    Artificial Sequence

<220>
<223>    Synthetic Sequence


<400>    51
cagggctaga ccttcgttat                                                    20


<210>    52
<211>    20
<212>    DNA
<213>    Artificial Sequence

<220>
<223>    Synthetic Sequence


<400>    52
atgcagtgta gcatggggag                                                    20


<210>    53
<211>    20
<212>    DNA

<213>    Artificial Sequence

<220>
<223>    Synthetic Sequence


<400>    53
agtctttgga caagatgccc                                                    20


<210>    54
<211>    20
<212>    DNA
<213>    Artificial Sequence

<220>
<223>    Synthetic Sequence


<400>    54
gaagaaagag aagagggctt                                                    20


<210>    55
<211>    20
<212>    DNA
<213>    Artificial Sequence

<220>
<223>    Synthetic Sequence


<400>    55
agcactttta tctcacccta                                                    20


<210>    56
<211>    20
<212>    DNA
<213>    Artificial Sequence

<220>
<223>    Synthetic Sequence


<400>    56
actgcctggg gttttccctt                                                    20


<210>    57
<211>    20
<212>    DNA
<213>    Artificial Sequence

<220>
<223>    Synthetic Sequence


<400>    57
gtgtcaacag ggtcactctg                                                    20


<210>    58

<211>    20
<212>    DNA
<213>    Artificial Sequence

<220>
<223>    Synthetic Sequence


<400>    58
aatttgcttt ggaaggacct                                                    20


<210>    59
<211>    20
<212>    DNA
<213>    Artificial Sequence

<220>
<223>    Synthetic Sequence


<400>    59
agattccaga gtgatggaat                                                    20


<210>    60
<211>    20
<212>    DNA
<213>    Artificial Sequence

<220>
<223>    Synthetic Sequence


<400>    60
agagatacag gagtccctgt                                                    20


<210>    61
<211>    20
<212>    DNA
<213>    Artificial Sequence

<220>
<223>    Synthetic Sequence


<400>    61
gagtaataag tctgctcttt                                                    20


<210>    62
<211>    20
<212>    DNA
<213>    Artificial Sequence

<220>
<223>    Synthetic Sequence


<400>    62
actttgagga ccttgaggaa                                                    20

<210> 63
<211> 20
<212> DNA
<213> Artificial Sequence

<220>
<223> Synthetic Sequence


<400> 63
actgtgggaa ctgtgggaga 20


<210> 64
<211> 20
<212> DNA
<213> Artificial Sequence

<220>
<223> Synthetic Sequence


<400> 64
caggcatatt ttcccatgta 20


<210> 65
<211> 20
<212> DNA
<213> Artificial Sequence

<220>
<223> Synthetic Sequence


<400> 65
atgtgatgct ggagagaaat 20


<210> 66
<211> 20
<212> DNA
<213> Artificial Sequence

<220>
<223> Synthetic Sequence


<400> 66
ggggttagtt tgtgttaact 20


<210> 67
<211> 20
<212> DNA
<213> Artificial Sequence

<220>
<223> Synthetic Sequence


<400> 67

acttacatca caggcatcac                                                    20

<210>    68
<211>    20
<212>    DNA
<213>    Artificial Sequence

<220>
<223>    Synthetic Sequence

<400>    68
atgccagatt cttcccagtg                                                    20

<210>    69
<211>    20
<212>    DNA
<213>    Artificial Sequence

<220>
<223>    Synthetic Sequence

<400>    69
acgaactgtt gggtggtgct                                                    20

<210>    70
<211>    20
<212>    DNA
<213>    Artificial Sequence

<220>
<223>    Synthetic Sequence

<400>    70
tttaatcgag cacatgcagg                                                    20

<210>    71
<211>    20
<212>    DNA
<213>    Artificial Sequence

<220>
<223>    Synthetic Sequence

<400>    71
tgatggatct gatggatact                                                    20

<210>    72
<211>    20
<212>    DNA
<213>    Artificial Sequence

<220>
<223>    Synthetic Sequence

<400>    72
aagaagggct ggtttgttct                                                    20


<210>    73
<211>    20
<212>    DNA
<213>    Artificial Sequence

<220>
<223>    Synthetic Sequence


<400>    73
acctgcagga actgaaacaa                                                    20


<210>    74
<211>    20
<212>    DNA
<213>    Artificial Sequence

<220>
<223>    Synthetic Sequence


<400>    74
ttgactccca tggtaacctg                                                    20


<210>    75
<211>    20
<212>    DNA
<213>    Artificial Sequence

<220>
<223>    Synthetic Sequence


<400>    75
atgaggttac tcagagccag                                                    20


<210>    76
<211>    20
<212>    DNA
<213>    Artificial Sequence

<220>
<223>    Synthetic Sequence


<400>    76
cacttgagtt caggagagct                                                    20


<210>    77
<211>    20
<212>    DNA
<213>    Artificial Sequence

<220>

<223>    Synthetic Sequence

<400>    77
cttcacttcc ctcctttcca                                                    20

<210>    78
<211>    20
<212>    DNA
<213>    Artificial Sequence

<220>
<223>    Synthetic Sequence

<400>    78
cactgccctc aagtccttac                                                    20

<210>    79
<211>    20
<212>    DNA
<213>    Artificial Sequence

<220>
<223>    Synthetic Sequence

<400>    79
caaactcacc aaatgtccac                                                    20

<210>    80
<211>    20
<212>    DNA
<213>    Artificial Sequence

<220>
<223>    Synthetic Sequence

<400>    80
ttctttggtt gtggtggttg                                                    20

<210>    81
<211>    20
<212>    DNA
<213>    Artificial Sequence

<220>
<223>    Synthetic Sequence

<400>    81
tagtgttggg gcataacacc                                                    20

<210>    82
<211>    20
<212>    DNA
<213>    Artificial Sequence

<220>
<223>    Synthetic Sequence


<400>    82
gtggcatttg gagtccatga                                                    20


<210>    83
<211>    20
<212>    DNA
<213>    Artificial Sequence

<220>
<223>    Synthetic Sequence


<400>    83
ctcagtactt ggtctcctgt                                                    20


<210>    84
<211>    20
<212>    DNA
<213>    Artificial Sequence

<220>
<223>    Synthetic Sequence


<400>    84
acctcttgag gggtaacaaa                                                    20


<210>    85
<211>    20
<212>    DNA
<213>    Artificial Sequence

<220>
<223>    Synthetic Sequence


<400>    85
ccctgaatac tgagcaaagc                                                    20


<210>    86
<211>    20
<212>    DNA
<213>    Artificial Sequence

<220>
<223>    Synthetic Sequence


<400>    86
gggcaagtgt gtgaagtgtg                                                    20


<210>    87
<211>    20

<212>    DNA
<213>    Artificial Sequence

<220>
<223>    Synthetic Sequence


<400>    87
acacagtaaa gacccaagta                                                    20


<210>    88
<211>    20
<212>    DNA
<213>    Artificial Sequence

<220>
<223>    Synthetic Sequence


<400>    88
tgtacatttc caggttcttc                                                    20


<210>    89
<211>    20
<212>    DNA
<213>    Artificial Sequence

<220>
<223>    Synthetic Sequence


<400>    89
ttggccagct tgtcctgagt                                                    20


<210>    90
<211>    20
<212>    DNA
<213>    Artificial Sequence

<220>
<223>    Synthetic Sequence


<400>    90
ggccaagatt gcatccagtc                                                    20


<210>    91
<211>    21
<212>    DNA
<213>    Artificial Sequence

<220>
<223>    Synthetic Sequence


<400>    91
tacctatgcc tcatgtagaa a                                                  21

<210>	92
<211>	21
<212>	DNA
<213>	Artificial Sequence

<220>
<223>	Synthetic Sequence


<400>	92
atcgtcaggt tctgggaccg t	21


<210>	93
<211>	21
<212>	DNA
<213>	Artificial Sequence

<220>
<223>	Synthetic Sequence


<400>	93
cagaagagag agagtagtag a	21


<210>	94
<211>	21
<212>	DNA
<213>	Artificial Sequence

<220>
<223>	Synthetic Sequence


<400>	94
gaatgtttaa ggtatagttt a	21


<210>	95
<211>	21
<212>	DNA
<213>	Artificial Sequence

<220>
<223>	Synthetic Sequence


<400>	95
tgtttcagca ggggttgaag c	21


<210>	96
<211>	21
<212>	DNA
<213>	Artificial Sequence

<220>
<223>	Synthetic Sequence


<400>	96
ctatacccta gacttattcc t	21

<210>	97
<211>	21
<212>	DNA
<213>	Artificial Sequence

<220>
<223>	Synthetic Sequence


<400>	97
gtttctttct acagaataga g	21


<210>	98
<211>	21
<212>	DNA
<213>	Artificial Sequence

<220>
<223>	Synthetic Sequence


<400>	98
ataggttaac aaagatattc a	21


<210>	99
<211>	21
<212>	DNA
<213>	Artificial Sequence

<220>
<223>	Synthetic Sequence


<400>	99
ctacaaaata ggtgacataa c	21


<210>	100
<211>	21
<212>	DNA
<213>	Artificial Sequence

<220>
<223>	Synthetic Sequence


<400>	100
ttaaaatggc gcaaataaat t	21


<210>	101
<211>	21
<212>	DNA
<213>	Artificial Sequence

<220>
<223>	Synthetic Sequence

<400>    101
ttttatgagt ctgccaggaa t                                                21


<210>    102
<211>    21
<212>    DNA
<213>    Artificial Sequence

<220>
<223>    Synthetic Sequence


<400>    102
gaagaaacaa ttttcactgg g                                                21


<210>    103
<211>    21
<212>    DNA
<213>    Artificial Sequence

<220>
<223>    Synthetic Sequence


<400>    103
caggaaggga gctagtgagc t                                                21


<210>    104
<211>    21
<212>    DNA
<213>    Artificial Sequence

<220>
<223>    Synthetic Sequence


<400>    104
cctctcctgc tgatgatccc c                                                21


<210>    105
<211>    21
<212>    DNA
<213>    Artificial Sequence

<220>
<223>    Synthetic Sequence


<400>    105
tgggccgcac gtgtgagtgc c                                                21


<210>    106
<211>    21
<212>    DNA
<213>    Artificial Sequence

<220>
<223>    Synthetic Sequence

<400>    106
aggaaacaag cccatgttcc c                                                21

<210>    107
<211>    21
<212>    DNA
<213>    Artificial Sequence

<220>
<223>    Synthetic Sequence

<400>    107
catggcagaa aacagaagac a                                                21

<210>    108
<211>    21
<212>    DNA
<213>    Artificial Sequence

<220>
<223>    Synthetic Sequence

<400>    108
aacagtctct gtgatagggc a                                                21

<210>    109
<211>    21
<212>    DNA
<213>    Artificial Sequence

<220>
<223>    Synthetic Sequence

<400>    109
gggaaaatca gacggatatt c                                                21

<210>    110
<211>    21
<212>    DNA
<213>    Artificial Sequence

<220>
<223>    Synthetic Sequence

<400>    110
atttctgttc ttccctcact t                                                21

<210>    111
<211>    21
<212>    DNA
<213>    Artificial Sequence

<220>
<223>    Synthetic Sequence


<400>    111
taataactgt gtagttataa c                                                21


<210>    112
<211>    21
<212>    DNA
<213>    Artificial Sequence

<220>
<223>    Synthetic Sequence


<400>    112
gttcagatta gctcttaact a                                                21


<210>    113
<211>    21
<212>    DNA
<213>    Artificial Sequence

<220>
<223>    Synthetic Sequence


<400>    113
taaacttcct atttattgtc t                                                21


<210>    114
<211>    21
<212>    DNA
<213>    Artificial Sequence

<220>
<223>    Synthetic Sequence


<400>    114
ataactaatg ccagggtgag t                                                21


<210>    115
<211>    21
<212>    DNA
<213>    Artificial Sequence

<220>
<223>    Synthetic Sequence


<400>    115
ctccacgccg gtagggttag a                                                21


<210>    116
<211>    21
<212>    DNA

<213>    Artificial Sequence

<220>
<223>    Synthetic Sequence


<400>    116
agtttgtagt gtctttccag a                                                21


<210>    117
<211>    21
<212>    DNA
<213>    Artificial Sequence

<220>
<223>    Synthetic Sequence


<400>    117
tattttccag atgttccaat a                                                21


<210>    118
<211>    21
<212>    DNA
<213>    Artificial Sequence

<220>
<223>    Synthetic Sequence


<400>    118
gactatgaga ctactactgc a                                                21


<210>    119
<211>    21
<212>    DNA
<213>    Artificial Sequence

<220>
<223>    Synthetic Sequence


<400>    119
ccttagcctc cttttcacac a                                                21


<210>    120
<211>    21
<212>    DNA
<213>    Artificial Sequence

<220>
<223>    Synthetic Sequence


<400>    120
agagaagaga aaatagggga a                                                21


<210>    121

<211>    21
<212>    DNA
<213>    Artificial Sequence

<220>
<223>    Synthetic Sequence


<400>    121
acacggacaa gttctccgtg a                                                21


<210>    122
<211>    21
<212>    DNA
<213>    Artificial Sequence

<220>
<223>    Synthetic Sequence


<400>    122
aacagtgggg cagttaggat t                                                21


<210>    123
<211>    21
<212>    DNA
<213>    Artificial Sequence

<220>
<223>    Synthetic Sequence


<400>    123
cacaagatag atagataaga t                                                21


<210>    124
<211>    21
<212>    DNA
<213>    Artificial Sequence

<220>
<223>    Synthetic Sequence


<400>    124
tttccaggtt ttgattgagg t                                                21


<210>    125
<211>    21
<212>    DNA
<213>    Artificial Sequence

<220>
<223>    Synthetic Sequence


<400>    125
cagatgccag aaaggagact g                                                21

<210>    126
<211>    21
<212>    DNA
<213>    Artificial Sequence

<220>
<223>    Synthetic Sequence


<400>    126
gaggagaagc ggcgataatc t                                                21


<210>    127
<211>    21
<212>    DNA
<213>    Artificial Sequence

<220>
<223>    Synthetic Sequence


<400>    127
gtagaaggct aaatagttac c                                                21


<210>    128
<211>    21
<212>    DNA
<213>    Artificial Sequence

<220>
<223>    Synthetic Sequence


<400>    128
ttataactaa gattctggcc c                                                21


<210>    129
<211>    21
<212>    DNA
<213>    Artificial Sequence

<220>
<223>    Synthetic Sequence


<400>    129
tctctttgca ccccaggcat g                                                21


<210>    130
<211>    21
<212>    DNA
<213>    Artificial Sequence

<220>
<223>    Synthetic Sequence


<400>    130

agataacaat aattattact t                                                21

<210>    131
<211>    21
<212>    DNA
<213>    Artificial Sequence

<220>
<223>    Synthetic Sequence

<400>    131
cccccctggcc aggcagggcc g                                               21

<210>    132
<211>    21
<212>    DNA
<213>    Artificial Sequence

<220>
<223>    Synthetic Sequence

<400>    132
agtagcacga acaaaacaag t                                                21

<210>    133
<211>    21
<212>    DNA
<213>    Artificial Sequence

<220>
<223>    Synthetic Sequence

<400>    133
gagaaaaatc aggatagagg t                                                21

<210>    134
<211>    21
<212>    DNA
<213>    Artificial Sequence

<220>
<223>    Synthetic Sequence

<400>    134
ctccacaagc agatgatcaa g                                                21

<210>    135
<211>    21
<212>    DNA
<213>    Artificial Sequence

<220>
<223>    Synthetic Sequence

<400>    135
aagttttgta gaaaactaaa t                                                21

<210>    136
<211>    21
<212>    DNA
<213>    Artificial Sequence

<220>
<223>    Synthetic Sequence

<400>    136
tactgtggga taactgacgg c                                                21

<210>    137
<211>    27
<212>    DNA
<213>    Artificial Sequence

<220>
<223>    Synthetic Sequence

<400>    137
gaggctattg gatttcattt ctagagt                                          27

<210>    138
<211>    27
<212>    DNA
<213>    Artificial Sequence

<220>
<223>    Synthetic Sequence

<400>    138
gcactcacga ggtcacgagg tgtgggt                                          27

<210>    139
<211>    27
<212>    DNA
<213>    Artificial Sequence

<220>
<223>    Synthetic Sequence

<400>    139
ctttcttaaa catagaatct ataggat                                          27

<210>    140
<211>    27
<212>    DNA
<213>    Artificial Sequence

<220>

<223>    Synthetic Sequence

<400>    140
gaacagtgca aggataggtg tgggggt                                          27

<210>    141
<211>    27
<212>    DNA
<213>    Artificial Sequence

<220>
<223>    Synthetic Sequence

<400>    141
tggtgccccg ggtttacact taagaat                                          27

<210>    142
<211>    27
<212>    DNA
<213>    Artificial Sequence

<220>
<223>    Synthetic Sequence

<400>    142
cttcatctat aggagcctcc agtgagt                                          27

<210>    143
<211>    27
<212>    DNA
<213>    Artificial Sequence

<220>
<223>    Synthetic Sequence

<400>    143
ggccttgagt gaggagaagg caggagt                                          27

<210>    144
<211>    27
<212>    DNA
<213>    Artificial Sequence

<220>
<223>    Synthetic Sequence

<400>    144
ggtgaagtac atattctcat atggagt                                          27

<210>    145
<211>    27
<212>    DNA
<213>    Artificial Sequence

<220>
<223>    Synthetic Sequence


<400>    145
gggctcagtt ttcccaccag tgggggt                                          27


<210>    146
<211>    27
<212>    DNA
<213>    Artificial Sequence

<220>
<223>    Synthetic Sequence


<400>    146
atacgttttg acggccaata gttgaat                                          27


<210>    147
<211>    27
<212>    DNA
<213>    Artificial Sequence

<220>
<223>    Synthetic Sequence


<400>    147
acctatgatg tgatagtttg tttgggt                                          27


<210>    148
<211>    27
<212>    DNA
<213>    Artificial Sequence

<220>
<223>    Synthetic Sequence


<400>    148
taagacctct taggaagtag aatgaat                                          27


<210>    149
<211>    27
<212>    DNA
<213>    Artificial Sequence

<220>
<223>    Synthetic Sequence


<400>    149
tttgagaggc aggggcacca gctgggt                                          27


<210>    150
<211>    27

<212>    DNA
<213>    Artificial Sequence

<220>
<223>    Synthetic Sequence


<400>    150
tggaagagtg gaaaaaggtg gaagagt                                          27


<210>    151
<211>    27
<212>    DNA
<213>    Artificial Sequence

<220>
<223>    Synthetic Sequence


<400>    151
tctgcagaac aggcgcccag tcagagt                                          27


<210>    152
<211>    27
<212>    DNA
<213>    Artificial Sequence

<220>
<223>    Synthetic Sequence


<400>    152
tgtagaattt ttaactgtta acaggat                                          27


<210>    153
<211>    27
<212>    DNA
<213>    Artificial Sequence

<220>
<223>    Synthetic Sequence


<400>    153
cagccaatgg tgtaataagc tgtgggt                                          27


<210>    154
<211>    27
<212>    DNA
<213>    Artificial Sequence

<220>
<223>    Synthetic Sequence


<400>    154
taaagagact caggagagaa gaagaat                                          27

<210>    155
<211>    27
<212>    DNA
<213>    Artificial Sequence

<220>
<223>    Synthetic Sequence


<400>    155
caagggaggt gcctggttgc ccagagt                                          27


<210>    156
<211>    27
<212>    DNA
<213>    Artificial Sequence

<220>
<223>    Synthetic Sequence


<400>    156
agtctatttt gattgttttt agggagt                                          27


<210>    157
<211>    27
<212>    DNA
<213>    Artificial Sequence

<220>
<223>    Synthetic Sequence


<400>    157
gcatcactga agaatcagcc taagagt                                          27


<210>    158
<211>    27
<212>    DNA
<213>    Artificial Sequence

<220>
<223>    Synthetic Sequence


<400>    158
gattttaatc ataactgcat gaagggt                                          27


<210>    159
<211>    27
<212>    DNA
<213>    Artificial Sequence

<220>
<223>    Synthetic Sequence


<400>    159
gtcacaagtt tctgtttctt ggtggat                                          27

<210> 160
<211> 27
<212> DNA
<213> Artificial Sequence

<220>
<223> Synthetic Sequence

<400> 160
tccatctctg aaatgtggat ggagaat 27

<210> 161
<211> 27
<212> DNA
<213> Artificial Sequence

<220>
<223> Synthetic Sequence

<400> 161
taaagggtgc ttttcttatt atagaat 27

<210> 162
<211> 27
<212> DNA
<213> Artificial Sequence

<220>
<223> Synthetic Sequence

<400> 162
tttgggagtg gagagatttg ggggagt 27

<210> 163
<211> 27
<212> DNA
<213> Artificial Sequence

<220>
<223> Synthetic Sequence

<400> 163
aggctctcag ggaatgagag gagggat 27

## Description

### [Technical Field]

The present invention relates to a composition for inducing death of cells having genomic sequence variations, comprising a nuclease and a cleavaging agent, and a method of inducing death of cells having genomic sequence variations.

### [Background Art]

Cells having damaged genes or genomes cause problems associated with survival or functions of organisms or organs thereof. There may be various methods of selectively inducing death of these cells. However, most of these methods additionally cause damage to normal cells, and thus are inapplicable to clinical use.

Cancer cells are the most representative cells having damaged genes or genomes that cause problems with the survival or functions of organisms or organs thereof. Although DNA damage is present in a very small portion compared to the human genome, DNA damage to proto-oncogenes or suppressor genes eventually increases the likelihood of the onset of cancer (Molecular Cell Biology. 4th edition. Lodish H., Berk A., Zipursky S.L., et al. New York: W. H. Freeman; 2000, "Section 12.4 DNA Damage and Repair and Their Role in Carcinogenesis").

Research and analysis on cancer-specific mutations are considered the main basis for the development of therapeutic agents for cancer. For example, it was possible to develop therapeutic agents targeting specific genetic mutations and to verify the correlation between mutation profiles and drug responsiveness through research on cancer mutations.

Cancer is caused by the accumulation of genetic mutations, which are inherited through germ cells or acquired in somatic cells during the cell cycle. Changes in these oncogenes, tumor suppressor genes and DNA repair genes cause cells to lose growth and regulatory mechanisms and thus to develop cancer.

In cancers caused by this process, a number of phenomena in which new DNA sequences not found in normal cells are inserted (Insertion: IN) or in which part of the DNA of normal cells is deleted (Deletion: Del) have been observed. The specific insertion or deletion DNA (IN/DEL) occurring in such DNA of cancer cells pertains to a DNA sequence that does not exist in normal cells, thus being useful as a differentiated attack target between normal cells and cancer cells.

Meanwhile, DNA double-stranded break (DSB) is one of the most severe forms of damage at the cellular level. Damaged DNA is repaired by non-homologous end joining and homologous recombination, whereas DNA that cannot be repaired may lead to damage or rearrangement of genetic information, causing cell death.

CRISPR/Cas is a gene-editing tool using RNA guide and is capable of introducing double-stranded (or single-stranded) breaks into specific positions of the genome by matching the guide RNA sequence to the genomic DNA sequence using the bacteria-induced endonuclease Cas9 (or mutant nickase) and guide RNA. CRISPR/Cas-mediated gene knockout is expected to be more efficient than RNA interference-mediated gene knockdown and provides a useful experimental tool for gene function research.

In research it has been reported that the CRISPR-Cas system can work in mammalian cells, and gene-editing techniques derived from adaptive immunity of microorganisms include Cas9 (CRISPR associated protein 9: RNA-guided DNA endonuclease enzyme) and guide RNA (gRNA). Guide RNA includes crRNA (CRISPR RNA) and tracrRNA (trans-activating crRNA), and binds to Cas9 and guides the same to the target genomic sequence through base pairing to the target sequence to form a double-stranded break (DSB). The only criterion for defining the target sequence is whether or not a protospacer adjacent motif (PAM) is present, and the sequence of the protospacer adjacent motif (PAM) differs depending on the Cas protein that recognizes the same. For example, it is known that Cas9 derived from *S. pyogenes* is 5'-NGG-3' (wherein N is A, T, G or C); Cas9 derived from *S. thermophilus* is 5'-NNAGAAW-3'; and Cas9 derived from *C*. *jejuni* is 5'-NNNNRYAC-3'. The PAM may be used for gene editing because the sequences are arranged at regular intervals on the human genome.

Meanwhile, it has been reported that the CRISPR/Cas system can be applied to human cancer therapy (Oncotarget. 2016 Mar 15;7(11):12305-17). However, this suggests that modification or deletion of one or more portions of the genome may increase the likelihood of providing potent therapeutics for cancer, based on a plurality of genetic mutations that are correlated with the onset of cancer.

Under this technical background, the present inventors found that cells having genomic sequence variations such as cancer cells have an inherent In/Del sequences thereof, also found that it was enabled to induce death of cells having genomic sequence variations from plural DNA DSBs (double strand breaks) in certain DNA site of cells, which are derived from cleavaging agent(s) and a nuclease produced on the basis of In/Del sequences, so that the present invention was completed based on this finding.

### [Disclosure]

### [Technical Problem]

Therefore, the present invention has been made in view of the above problems, and it is one object of the present invention to provide a composition for inducing death of cells having genomic sequence variations comprising a nuclease and a cleavaging agent.

It is another object of the present invention to provide a composition for treating cancer comprising a nuclease and a cleavaging agent.

It is another object of the present invention to provide a method for inducing death of cells having genomic sequence variations comprising a nuclease and a cleavaging agent.

It is another object of the present invention to provide a method for treating cancer using a nuclease and a cleavaging agent.

### [Technical Solution]

In accordance with one aspect of the present invention, the above and other objects can be accomplished by the provision of a composition for inducing death of cells having genomic sequence variations comprising a nuclease and a cleavaging agent that specifically recognizes a nucleic acid sequence comprising a mutant sequence specific to cells having genomic sequence variations.

In accordance with another aspect, provided is a composition for treating a cancer comprising a nuclease and a cleavaging agent that specifically recognizes a nucleic acid sequence comprising an insertion and/or deletion specific to the cancer.

In accordance with another aspect, provided is a method for inducing death of cells having genomic sequence variations, comprising treating cells having genomic sequence variations with a nuclease and a cleavaging agent that specifically recognizes a nucleic acid sequence comprising a mutant sequence specific to cells having genomic sequence variations.

In accordance with another aspect, provided is a method for inducing death of cells having genomic sequence variations comprising: performing whole-genome sequencing (WGS) on cells having genomic sequence variations and normal cells;
comparing the resulting WGS data between cells having genomic sequence variations and the normal cells to select a mutant sequence specific to cells having genomic sequence variations;
producing a cleavaging agent that recognizes the selected mutant sequence;
preparing a composition comprising a cleavaging agent and a nuclease; and
applying the composition to cells having genomic sequence variations.

In accordance with another aspect, provided is a method for inducing death of cells having genomic sequence variations comprising:
performing whole-genome sequencing (WGS) on cells having genomic sequence variations and normal cells;
comparing the resulting WGS data between cells having genomic sequence variations and the normal cells to select In/Del(s) specific to cells having genomic sequence variations;
producing a cleavaging agent that recognizes selected In/Dels;
preparing a composition comprising a cleavaging agent and a nuclease; and
applying the composition comprising the nuclease and the cleavaging agent to cells having genomic sequence variations.

In accordance with another aspect, provided is a method of treating cancer comprising administering a nuclease and a cleavaging agent that specifically recognizes a nucleic acid sequence comprising a mutant sequence specific to cells having genomic sequence variations and a nuclease to a subject.

In accordance with another aspect, provided is a method of treating cancer comprising treating cells having genomic sequence variations with a vector comprising an expression cassette of a cleavaging agent that specifically recognizes a nucleic acid sequence comprising an insertion and/or deletion specific to cells having genomic sequence variations and a nuclease.

In accordance with another aspect, provided is a composition for patient-specific cancer therapy comprising a nuclease and a cleavaging agent that specifically recognizes a nucleic acid sequence comprising an insertion and/or deletion specific to cancer cells of the patient.

In accordance with another aspect, provided is a patient-specific cancer therapy comprising: selecting In/Del(s) specific to the cancer cells from cancer cells of a patient; producing a cleavaging agent recognizing In/Del(s); and delivering a composition comprising a nuclease and the cleavaging agent to the patient.

### [Description of Drawings]

FIG. 1 shows the result of detection as to whether or not cells are induced to death, when a plurality of DSBs (double-stranded breaks) of DNA occur simultaneously;
FIG. 2 shows the result of detection as to whether or not DNA is cleaved by a CRIPSR system using guide RNA;
FIG. 3 shows cell growth detected through a colony forming assay after transfection of 30 specific RNP (ribonucleotide protein) complexes into colorectal cancer cells and osteosarcoma cells to induce DNA DSBs;
FIG. 4 shows cell infection of AAV measured using immunofluorescence and flow cytometry;
FIG. 5 shows the result of detection of transfection of AAV particles through immunofluorescence;
FIG. 6 shows U2OS-specific crRNA-dependent cell death detected using 30 U20S-cell-line-specific crRNAs;
FIG. 7 shows the results of detection as to whether or not a U20S-cell-specific saCAS9 AAV system operates and whether or not cell death occurs in U2OS cells;
FIG. 8 shows cell viability (%) measured based on cell death by cancer-specific In/Del;
FIG. 9 shows the result of detection as to whether or not AAV-dependent cell death is induced only in crRNA-specific cell lines;
FIG. 10 shows the result of detection as to whether or not selective cancer cell death occurs in glioblastoma;
FIG. 11 shows the result of detection of the difference in cell death between the use of AAV particles comprising an ATM kinase inhibitor and the use of AAV particles not comprising an ATM kinase inhibitor; and
FIG. 12 shows the result of detection of the effect of lung-cancer-specific In/Del-induced cell death (CINDELA).

### [Best Mode]

Unless defined otherwise, all technical and scientific terms used herein have the same meanings as appreciated by those skilled in the field to which the present invention pertains. In general, the nomenclature used herein is well-known in the art and is ordinarily used.

In one aspect, the present invention is directed to a composition for inducing death of cells having genomic sequence variations comprising a nuclease and a cleavaging agent that specifically recognizes a nucleic acid sequence comprising a mutant sequence specific to cells having genomic sequence variations.

In another aspect, the present invention is directed to a composition for treating a cancer comprising a nuclease and a cleavaging agent that specifically recognizes a nucleic acid sequence comprising an insertion and/or deletion specific to the cancer.

In another aspect, the present invention is directed to a method for inducing death of cells having genomic sequence variations, comprising treating cells having genomic sequence variations with a nuclease and a cleavaging agent that specifically recognizes a nucleic acid sequence comprising a mutant sequence specific to cells having genomic sequence variations.

As used herein, the term "cells having genomic sequence variations" (also called "mutated cells" or "cells having a genetic mutations") refers to cells that are imparted with different activity from that of normal cells by genetic mutations, and may, for example, refer to cells that are in the state of onset of a disease due to genetic mutations, specifically, cancer cells.

The cancer is, for example, melanoma, small-cell lung cancer, non-small-cell lung cancer, glioma, liver cancer, thyroid tumor, gastric cancer, ovarian cancer, bladder cancer, lung cancer, colorectal cancer, breast cancer, prostate cancer, glioblastoma, endometrial cancer, kidney cancer, colon cancer, pancreatic cancer, esophageal carcinoma, head and neck cancer, mesothelioma, sarcoma, osteosarcoma, bile duct cancer, or epidermal cancer, but is not limited thereto.

A phenomenon in which a new DNA sequence not found in normal cells is inserted, called "Insertion" (IN), or in which a part of the DNA of normal cells is deleted, called "deletion (Del)", is observed in cancer cells, and a DNA sequence specifically inserted into or deleted from respective cancer cells may be present therein.

Cells have a DNA damage repair mechanism for repairing damage to DNA when a DSB (double-stranded break) of the DNA in cells occurs. However, the DNA damage repair mechanism effectively repairs damage to DNA when the number of double-stranded breaks is small, but causes death when the number of double-stranded breaks is great. Bacteria can be killed by a single double-stranded break, but more multiple DSBs are required in order to induce death of animal cells.

According to the present invention, based on the facts described above, the inventors found multiple In/Dels of cells having genomic sequence variations, for example, cancer cells, and produced multiple cleavaging agents capable of recognizing multiple In/Dels, and finally induced specific death of cells having genomic sequence variations, for example, cancer cells, using a nuclease and multiple cleavaging agents.

The nuclease, which is a means of achieving a DNA double-stranded break, may be a restriction enzyme, a zinc finger nuclease (ZNFN), a transcriptional activator-like effector nuclease (TALEN), or a Cas protein, or a nucleic acid encoding the same, but is not limited thereto. The Cas protein may be Cas3, Cas9, Cpf1 (CRISPR from *Prevotella and Francisella* 1), Cas6, C2c12, or C2c2, but is not limited thereto.

The Cas protein may be derived from a microorganism genus comprising an ortholog of a Cas protein selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus (Staphylococcus aureus), Nitratifractor, Corynebacterium* and *Campylobacter,* and the Cas protein is isolated therefrom or recombined.

In another aspect, the present invention is directed to a method for inducing death of cells having genomic sequence variations comprising: performing whole-genome sequencing (WGS) on cells having genomic sequence variations and normal cells; comparing the resulting WGS data between cells having genomic sequence variations and the normal cells to select a mutant sequence specific to cells having genomic sequence variations; producing a cleavaging agent that recognizes the selected mutant sequence; preparing a composition comprising a cleavaging agent and a nuclease; and applying the composition to cells having genomic sequence variations.

In another aspect, the present invention is directed to a method for inducing death of cells having genomic sequence variations comprising: performing whole-genome sequencing (WGS) on cells having genomic sequence variations, for example, cancer cells, and normal cells; comparing the resulting WGS data between cells having genomic sequence variations and the normal cells to select multiple In/Del(s) specific to cells having genomic sequence variations; producing a cleavaging agent that recognizes selected In/Dels; preparing a composition comprising a cleavaging agent and a nuclease; and applying the composition comprising the nuclease and the cleavaging agent to cells having genomic sequence variations.

Mapping of In/Del, which is a target of cleavaging agent, can be performed through WGS (whole-genome sequencing), or subtractive hybridization and sequencing. In the case where the derived In/Del is an insertion found in cancer, guide RNA is prepared immediately, and in the case where the derived In/Del is a deletion found in cancer, a break point is mapped and then a guide RNA including the break point is produced.

As used herein, the term "WGS (whole-genome sequencing" refers to a method of reading a genome in various depth of 10x, 20x and 40x using full-length genome sequences by next-generation sequencing. As used herein, the term "next-generation sequencing" refers to technology that includes fragmenting a full-length genome in a chip-based and PCR-based paired end format and sequencing the fragment at a very high speed based on chemical hybridization.

Subtractive hybridization is a method used for cloning genes with differences in expression between several tissues or cells. Genes specific to the DNA sample of cells to be tested can be detected. The DNA of the cells to be tested is modified into single-stranded DNA and then annealed. By adjusting the annealing conditions, the DNA sequence specific to the cells to be tested can be separated into double-stranded DNA.

The nucleic acid sequence including In/Del specific to cancer cells, which are a type of cell having genetic mutation, for example, may comprise a gene site where DSB of DNA is induced in a nucleic acid sequence by a nuclease targeting In/Del, and a sequence in a nucleic acid sequence that is specifically recognized by a nuclease, for example, a nucleic acid sequence having a length of about 17 bp to 23 bp adjacent to the 5' end and/or 3' end of the PAM sequence recognized by a Cas9 protein, when the nuclease is Cas9.

The nucleic acid sequence including In/Del specific to cancer cells, which are cells having genomic sequence variations, is represented by the nucleic acid sequence of the strand where the PAM sequence is located, among two DNA strands of the corresponding sequence site. In this case, since the DNA strand to which the guide RNA actually binds is the strand complementary to the strand where the PAM sequence is located, the targeting sequence included in the guide RNA has the same nucleic acid sequence as the nucleic acid sequence including In/Del, except that T is changed to U due to the characteristics of RNA.

When the Cas9 protein is derived from *Streptococcus pyogenes,* the PAM sequence may be 5'-NGG-3' (wherein N is A, T, G, or C), and the nucleic acid sequence including In/Del specific to cells having genomic sequence variations may be a gene site located adjacent to the 5' end and/or 3' end of the 5'-NGG-3' sequence in the sequence, for example, a gene site having a maximum length of about 50 bp or about 40 bp.

When the Cas9 protein is derived from *Streptococcus thermophilus,* the PAM sequence may be 5'-NNAGAAW-3' (wherein N is A, T, G, or C), and the nucleic acid sequence including In/Del specific to cells having genomic sequence variations may be a gene site located adjacent to the 5' end and/or the 3' end of the 5'-NNAGAAW-3' sequence in the sequence, for example, a gene site having a maximum length of about 50 bp or about 40 bp.

When the Cas9 protein is derived from *Staphylococcus aureus,* the PAM sequence may be 5'-NNGRRT-3' (wherein N is A, T, G, or C and R is A or G), and the nucleic acid sequence including In/Del specific to cells having genomic sequence variations may be a gene site located adjacent to the 5' end and/or 3' end of the 5'-NNAGAAW-3' sequence in the sequence, for example, a gene site having a maximum length of about 50 bp or about 40 bp.

When the Cas9 protein is derived from *Campylobacter jejuni,* the PAM sequence may be 5'-NNNNRYAC-3' (wherein N is A, T, G, or C, R is A or G, and Y is C or T), and the nucleic acid sequence including In/Del specific to cells having genomic sequence variations may be a gene site located adjacent to the 5' end and/or the 3' end of the 5'-NNNNRYAC-3' sequence in the sequence, for example, a gene site having a maximum length of about 50 bp or about 40 bp.

As used herein, the term "cleavaging agent" refers to a nucleotide sequence that enables recognizing and cleaving a modified and changed portion of a nucleic acid sequence of a cell having genomic sequence variations compared to a normal cell.

The cleavaging agent used herein should be present in a plural number with different sequences sufficient to induce death of cells compared to the nucleotide sequence of normal cells, preferably 1 to 30, more preferably 10 to 30, and still more preferably 16 to 30, but the number thereof may vary depending on the type of cells or cleavaging agents.

The cleavaging agent that specifically recognizes a nucleic acid sequence including In/Del specific to cells having genomic sequence variations, for example, cancer cells, may be, for example, guide RNA. The guide RNA may, for example, include at least one selected from the group consisting of CRISPR RNA (crRNA), trans-activating crRNA (tracrRNA), and single guide RNA (sgRNA), specifically, a double-stranded crRNA:tracrRNA complex comprising crRNA and tracrRNA bonded to each other, or single-stranded guide RNA (sgRNA) having crRNA or a portion thereof and tracrRNA or a portion thereof linked to each other by an oligonucleotide linker.

The guide RNA that specifically recognizes the nucleic acid sequence including In/Del specific to cells having genomic sequence variations means a nucleotide sequence having a sequence complementarity of at least 90%, at least 95%, at least 96%, at least 97%, at least 98%, at least 99%, or 100% with the nucleotide sequence of the strand complementary to the DNA strand where the PAM sequence is located, and can be linked to the nucleotide sequence of the complementary strand.

The guide RNA may be produced through the following steps: comparing the resulting WGS data between cancer cells and normal cells to select In/Del specific to the cancer cells, designing cancer-cell-specific guide RNA(s) that satisfies the conditions of guide RNA production based on the In/Del, and then setting an arbitrary order in consideration of the length of the In/Del site and designing the guide RNA homogeneously on all chromosomes to complete a final guide RNA combination.

The conditions for guide RNA production are as follows: (a) the length of the nucleotide sequence of the guide RNA excluding the PAM site is 20 base pairs; (b) the total proportion of guanine and cytosine present in the guide RNA is between 40% and 60%; (c) In/Del exists in the immediate front area near the PAM site; (d) the maximum homopolymer length is 4 base pairs or less; and (e) mapping that allows one mismatch to the resulting WGS data of normal cells is performed, there should be no mapping result.

The guide RNA produced based thereon may, for example, include at least one sequence selected from the group consisting of SEQ ID NOS: 1 to 163. The number of guide RNAs capable of inducing death of cancer cells may be multiple with different sequences, specifically about 1 to about 40, about 15 to about 25, or about 10 to about 20. The guide RNA may, for example, include at least one sequence selected from the group consisting of SEQ ID NOS: 1 to 30, at least one sequence selected from the group consisting of SEQ ID NOS: 31 to 60, at least one sequence selected from the group consisting of SEQ ID NOS: 61 to 90, at least one sequence selected from the group consisting of SEQ ID NOS: 91 to 120, at least one sequence selected from the group consisting of SEQ ID NOS: 121 to 136, and at least one sequence selected from the group consisting of SEQ ID NOS: 137 to 163.

In one embodiment, the cancer may be colorectal cancer, and a cleavaging agent that specifically recognizes a nucleic acid sequence including an insertion and/or deletion specific to the colorectal cancer may be guide RNA including at least one sequence selected from the group consisting of sequences represented by SEQ ID NOS: 1 to 30.

In one embodiment, the cancer may be osteosarcoma, and a cleavaging agent that specifically recognizes a nucleic acid sequence including an insertion and/or deletion specific to the osteosarcoma may be guide RNA including at least one sequence selected from the group consisting of sequences represented by SEQ ID NOS: 31 to 60 or guide RNA including at least one sequence selected from the group consisting of sequences represented by SEQ ID NOS: 91 to 120.

In one embodiment, a cleavaging agent that specifically recognizes a nucleic acid sequence including an insertion and/or deletion specific to normal cells may be guide RNA including at least one sequence selected from the group consisting of sequences represented by SEQ ID NOS: 61 to 90.

In one embodiment, the cancer may be glioblastoma, and a cleavaging agent that specifically recognizes a nucleic acid sequence including an insertion and/or deletion specific to the glioblastoma may be, for example, guide RNA including at least one sequence selected from the group consisting of sequences represented by SEQ ID NOS: 121 to 136.

In one embodiment, the cancer may be lung cancer, and a cleavaging agent that specifically recognizes a nucleic acid sequence including an insertion and/or deletion specific to the lung cancer may be guide RNA including at least one sequence selected from the group consisting of sequences represented by SEQ ID NOS: 137 to 163.

According to an embodiment of the present invention, cancer-specific In/Del was identified in cells of each of the colorectal cancer cell line HCT116, the osteosarcoma cell line U2OS, the glioblastoma cell line GBL-67, lung cancer tissue and the cell line REP1 obtained by immortalization of normal cells, guide RNA that specifically recognizes the cancer-specific In/Del was produced to induce DNA DSB, and then cell growth was observed. Specifically, the guide RNA that specifically recognizes In/Del of the colorectal cancer cell line HCT116 includes at least one sequence selected from the group consisting of SEQ ID NOS: 1 to 30, and the guide RNA that specifically recognizes In/Del of the osteosarcoma cell line U2OS includes at least one sequence selected from the group consisting of SEQ ID NOS: 31 to 60, or SEQ ID NOS: 91 to 120, the guide RNA that specifically recognizes In/Del of the glioblastoma cell line includes at least one sequence selected from the group consisting of SEQ ID NOS: 121 to 136, the guide RNA that specifically recognizes In/Del of the lung cancer cell line includes at least one sequence selected from the group consisting of SEQ ID NOS: 137 to 163, and the guide RNA that specifically recognizes In/Del of normal cell line REP1 includes at least one sequence selected from the group consisting of SEQ ID NOS: 61 to 90.

The cell-line-specific In/Del is detected through whole genome translation (WGS), and then guide RNA is designed to be included in the region of the PAM site that strongly binds to the corresponding region. It should be confirmed that the designed guide RNA is found to not cause a non-specific response to a normal human standard genome. Then, an arbitrary order is determined in consideration of the length of the In/Del site, and guide RNAs are designed to be evenly distributed throughout all chromosomes based on the order, to thereby complete a final multiple guide RNA combinations. Although 30 guide RNAs were used in this embodiment, the number of the guide RNAs may be adjusted depending on the type of cancer cells and the experimental method that causes DSBs.

The nuclease and cleavaging agent, for example, guide RNA, according to the present invention may be delivered, into cells, in the form of (a) multiple guide RNAs with different sequences and a vector including a nucleic acid sequence encoding a nuclease, for example, a Cas protein, (b) ribonucleoprotein (RNP) or RNA-guided engineered nuclease (RGEN) comprising multiple guide RNAs with different sequences and a nuclease, for example, a Cas protein, or (c) at least one guide RNA and mRNA encoded by a nuclease, for example, a Cas protein, but is not limited thereto.

In one embodiment, the vector may be a viral vector. The viral vector may be selected from negative-stranded RNA viruses such as retrovirus, adenovirus-parvovirus (e.g., adeno-associated virus (AAV)), coronavirus and orthomyxovirus (e.g. influenza virus), positive-stranded RNA viruses such as rhabdovirus (e.g., rabies and vesicular stomatitis virus), paramyxovirus (e.g., measles and Sendai), alphavirus and picornavirus, double-stranded DNA virus including herpesvirus (e.g., Herpes Simplex virus types 1 and 2, Epstein-Barr virus, cytomegalovirus) and adenovirus, pox virus (e.g., vaccinia, fowlpox or canarypox), and the like.

The vector may be delivered *in vivo* or into cells through electroporation, lipofection, viral vectors, or nanoparticles, as well as PTD (protein translocation domain) fusion protein methods.

In some cases, for example, at least one ATM (Ataxia telangiectasia mutated) inhibitor selected from the group consisting of caffeine, wortmannin, CP-466722, KU-55933, KU-60019 and KU-559403, at least one ATR (Ataxia telangiectasia and Rad-3 mutated) inhibitor selected from the group consisting of Schisandrin B, NU6027, NVP-BEZ235, VE-821, VE-822 (VX-970), AZ20 and AZD6738, or a DNA double-strand repair inhibitor of DNA-PKcs (DNA-dependent protein kinase catalytic subunit) may be further included to inhibit DNA double-strand repair in order to increase cell death efficiency through DNA double-stranded break.

In another aspect, the present invention is directed to a composition for treating cancer comprising a nuclease and a cleavaging agent that specifically recognizes a nucleic acid sequence comprising an insertion and/or deletion specific to the cancer. In another aspect, the present invention is directed to a method of treating cancer including administering a nuclease and a cleavaging agent that specifically recognizes a nucleic acid sequence comprising an insertion and/or deletion specific to the cancer to a subject.

In another aspect, the present invention is directed to a composition for patient-specific cancer therapy comprising a nuclease and a cleavaging agent that specifically recognize a nucleic acid sequence comprising an insertion and/or deletion specific for cancer cells of the patient.

As used herein, the term "patient-specific cancer" means that the inherent nature of a patient or the properties of the disease of the patient are fully considered so as to effectively treat cancer. The composition for patient-specific cancer therapy may be usefully utilized in selection of therapeutic agents and methods.

As used herein, the term "cells having genomic sequence variations" refers to cells that are imparted with different activity from that of normal cells by genetic modification, and may, for example, refer to cells that are in the state of onset of a disease due to genetic mutation, specifically, cancer cells.

The cancer is, for example, melanoma, small-cell lung cancer, non-small-cell lung cancer, glioma, liver cancer, thyroid tumor, gastric cancer, ovarian cancer, bladder cancer, lung cancer, colorectal cancer, breast cancer, prostate cancer, glioblastoma, endometrial cancer, kidney cancer, colon cancer, pancreatic cancer, esophageal carcinoma, head and neck cancer, mesothelioma, sarcoma, osteosarcoma, bile duct cancer, or epidermal cancer, but is not limited thereto.

A phenomenon in which a new DNA sequence not found in normal cells is inserted, called "Insertion" (IN), or in which a part of the DNA of normal cells is deleted, called "deletion (Del)", is observed in cancer cells, and a DNA sequence specifically inserted into or deleted from respective cancer cells may be present therein.

Cells have a DNA damage repair mechanism for repairing damage to DNA when a DSB (double-stranded break) of the DNA in cells occurs. However, the DNA damage repair mechanism effectively repairs damage to DNA when the number of double-stranded breaks is small, but causes death when the number of double-stranded breaks is great. Bacteria can be killed by a single double-stranded break, but more multiple DSBs are required in order to induce death of animal cells.

According to the present invention, based on the facts described above, the inventors found multiple In/Dels of cells having genomic sequence variations, for example, cancer cells, and produced multiple cleavaging agents capable of recognizing multiple In/Dels, and finally induced specific death of cells having genomic sequence variations, for example, cancer cells, using a nuclease and multiple cleavaging agents.

The nuclease, which is a means of achieving a DNA double-stranded break, may be a restriction enzyme, a zinc finger nuclease (ZNFN), a transcriptional activator-like effector nuclease (TALEN), or a Cas protein, or a nucleic acid encoding the same, but is not limited thereto. The Cas protein may be Cas3, Cas9, Cpf1 (CRISPR from *Prevotella and Francisella* 1), Cas6, C2c12, or C2c2, but is not limited thereto.

The Cas protein may be derived from a microorganism genus comprising an ortholog of a Cas protein selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus (Staphylococcus aureus), Nitratifractor, Corynebacterium* and *Campylobacter,* and the Cas protein is isolated therefrom or recombined.

The nucleic acid sequence including In/Del specific to cancer cells, which are a type of cell having genetic mutation, for example, may comprise a gene site where DSB of DNA is induced in a nucleic acid sequence by a nuclease targeting In/Del, and a sequence in a nucleic acid sequence that is specifically recognized by a nuclease, for example, a nucleic acid sequence having a length of about 17 bp to 23 bp adjacent to the 5' end and/or 3' end of the PAM sequence recognized by a Cas9 protein, when the nuclease is Cas9.

The nucleic acid sequence including In/Del specific to cancer cells, which are cells having genomic sequence variations, is represented by the nucleic acid sequence of the strand where the PAM sequence is located, among two DNA strands of the corresponding sequence site. In this case, since the DNA strand to which the guide RNA actually binds is the strand complementary to the strand where the PAM sequence is located, the targeting sequence included in the guide RNA has the same nucleic acid sequence as the nucleic acid sequence including In/Del, except that T is changed to U due to the characteristics of RNA.

When the Cas9 protein is derived from *Streptococcus pyogenes,* the PAM sequence may be 5'-NGG-3' (wherein N is A, T, G, or C), and the nucleic acid sequence including In/Del specific to cells having genomic sequence variations may be a gene site located adjacent to the 5' end and/or 3' end of the 5'-NGG-3' sequence in the sequence, for example, a gene site having a maximum length of about 50 bp or about 40 bp.

When the Cas9 protein is derived from *Streptococcus thermophilus,* the PAM sequence may be 5'-NNAGAAW-3' (wherein N is A, T, G, or C), and the nucleic acid sequence including In/Del specific to cells having genomic sequence variations may be a gene site located adjacent to the 5' end and/or the 3' end of the 5'-NNAGAAW-3' sequence in the sequence, for example, a gene site having a maximum length of about 50 bp or about 40 bp.

When the Cas9 protein is derived from *Staphylococcus aureus,* the PAM sequence may be 5'-NNGRRT-3' (wherein N is A, T, G, or C and R is A or G), and the nucleic acid sequence including In/Del specific to cells having genomic sequence variations may be a gene site located adjacent to the 5' end and/or 3' end of the 5'-NNAGAAW-3' sequence in the sequence, for example, a gene site having a maximum length of about 50 bp or about 40 bp.

When the Cas9 protein is derived from *Campylobacter jejuni,* the PAM sequence may be 5'-NNNNRYAC-3' (wherein N is A, T, G, or C, R is A or G, and Y is C or T), and the nucleic acid sequence including In/Del specific to cells having genomic sequence variations may be a gene site located adjacent to the 5' end and/or the 3' end of the 5'-NNNNRYAC-3' sequence in the sequence, for example, a gene site having a maximum length of about 50 bp or about 40 bp.

As used herein, the term "cleavaging agent" refers to a nucleotide sequence that enables recognizing and cleaving a modified and changed portion of a nucleic acid sequence of a cell having genomic sequence variations compared to a normal cell.

The cleavaging agent used herein should be present in a plural number with different sequences sufficient to induce death of cells compared to the nucleotide sequence of normal cells, preferably 1 to 30, more preferably 10 to 30, and still more preferably 16 to 30, but the number thereof may vary depending on the type of cells or cleavaging agents.

The cleavaging agent that specifically recognizes a nucleic acid sequence including In/Del specific to cells having genomic sequence variations, for example, cancer cells, may be, for example, guide RNA. The guide RNA may, for example, include at least one selected from the group consisting of CRISPR RNA (crRNA), trans-activating crRNA (tracrRNA), and single guide RNA (sgRNA), specifically, a double-stranded crRNA:tracrRNA complex comprising crRNA and tracrRNA bonded to each other, or single-stranded guide RNA (sgRNA) having crRNA or a portion thereof and tracrRNA or a portion thereof linked to each other by an oligonucleotide linker.

The guide RNA that specifically recognizes the nucleic acid sequence including In/Del specific to cells having genomic sequence variations means a nucleotide sequence having a sequence complementarity of at least 90%, at least 95%, at least 96%, at least 97%, at least 98%, at least 99%, or 100% with the nucleotide sequence of the strand complementary to the DNA strand where the PAM sequence is located, and can be linked to the nucleotide sequence of the complementary strand.

The guide RNA may be produced through the following steps: comparing the resulting WGS data between cancer cells and normal cells to select In/Del specific to the cancer cells, designing cancer-cell-specific guide RNA that satisfies the conditions of guide RNA production based on the In/Del, and then setting an arbitrary order in consideration of the length of the In/Del site and designing the guide RNA homogeneously on all chromosomes to complete a final guide RNA combination.

The conditions for guide RNA production are as follows: (a) the length of the nucleotide sequence of the guide RNA excluding the PAM site is 20 base pairs; (b) the total proportion of guanine and cytosine present in the guide RNA is between 40% and 60%; (c) In/Del exists in the immediate front area near the PAM site; (d) the maximum homopolymer length is 4 base pairs or less; and (e) mapping that allows one mismatch to the resulting WGS data of normal cells is performed, there should be no mapping result.

The guide RNA produced based thereon may, for example, include at least one sequence selected from the group consisting of SEQ ID NOS: 1 to 163. The number of guide RNAs capable of inducing death of cancer cells may be multiple with different sequences, specifically about 1 to about 40, about 15 to about 25, or about 10 to about 20. The guide RNA may, for example, include at least one sequence selected from the group consisting of SEQ ID NOS: 1 to 30, at least one sequence selected from the group consisting of SEQ ID NOS: 31 to 60, at least one sequence selected from the group consisting of SEQ ID NOS: 61 to 90, at least one sequence selected from the group consisting of SEQ ID NOS: 91 to 120, at least one sequence selected from the group consisting of SEQ ID NOS: 121 to 136, and at least one sequence selected from the group consisting of SEQ ID NOS: 137 to 163.

In one embodiment, the cancer may be colorectal cancer, and a cleavaging agent that specifically recognizes a nucleic acid sequence including an insertion and/or deletion specific to the colorectal cancer may be guide RNA including at least one sequence selected from the group consisting of sequences represented by SEQ ID NOS: 1 to 30.

In one embodiment, the cancer may be osteosarcoma, and a cleavaging agent that specifically recognizes a nucleic acid sequence including an insertion and/or deletion specific to the osteosarcoma may be guide RNA including at least one sequence selected from the group consisting of sequences represented by SEQ ID NOS: 31 to 60 or guide RNA including at least one sequence selected from the group consisting of sequences represented by SEQ ID NOS: 91 to 120.

In one embodiment, a cleavaging agent that specifically recognizes a nucleic acid sequence including an insertion and/or deletion specific to normal cells may be guide RNA including at least one sequence selected from the group consisting of sequences represented by SEQ ID NOS: 61 to 90.

In one embodiment, the cancer may be glioblastoma, and a cleavaging agent that specifically recognizes a nucleic acid sequence including an insertion and/or deletion specific to the glioblastoma may be, for example, guide RNA including at least one sequence selected from the group consisting of sequences represented by SEQ ID NOS: 121 to 136.

In one embodiment, the cancer may be lung cancer, and a cleavaging agent that specifically recognizes a nucleic acid sequence including an insertion and/or deletion specific to the lung cancer may be guide RNA including at least one sequence selected from the group consisting of sequences represented by SEQ ID NOS: 137 to 163.

The nuclease and cleavaging agent, for example, guide RNA, according to the present invention may be delivered, into cells, in the form of (a) multiple guide RNAs with different sequences and a vector including a nucleic acid sequence encoding a nuclease, for example, a Cas protein, (b) ribonucleoprotein (RNP) or RNA-guided engineered nuclease (RGEN) comprising multiple guide RNAs with different sequences and a nuclease, for example, a Cas protein, or (c) at least one guide RNA and mRNA encoded by a nuclease, for example, a Cas protein, but is not limited thereto.

In one embodiment, the vector may be a viral vector. The viral vector may be selected from negative-stranded RNA viruses such as retrovirus, adenovirus-parvovirus (e.g., adeno-associated virus (AAV)), coronavirus and orthomyxovirus (e.g. influenza virus), positive-stranded RNA viruses such as rhabdovirus (e.g., rabies and vesicular stomatitis virus), paramyxovirus (e.g., measles and Sendai), alphavirus and picornavirus, double-stranded DNA viruses including herpesvirus (e.g., Herpes Simplex virus types 1 and 2, Epstein-Barr virus, cytomegalovirus) and adenovirus, pox virus (e.g., vaccinia, fowlpox or canarypox), and the like.

The vector may be delivered *in vivo* or into cells through electroporation, lipofection, viral vectors, or nanoparticles, as well as PTD (protein translocation domain) fusion protein methods.

In some cases, a DNA double-strand repair inhibitor, for example, at least one ATM (Ataxia telangiectasia mutated) inhibitor selected from the group consisting of caffeine, wortmannin, CP-466722, KU-55933, KU-60019 and KU-559403, at least one ATR (Ataxia telangiectasia and Rad-3 mutated) inhibitor selected from the group consisting of Schisandrin B, NU6027, NVP-BEZ235, VE-821, VE-822 (VX-970), AZ20 and AZD6738, or DNA-PKcs (DNA-dependent protein kinase catalytic subunit) may be further included to inhibit DNA double-strand repair in order to increase cell death efficiency through DNA double-stranded break.

Moreover, in another aspect, the present invention is directed to a patient-specific cancer therapy comprising: selecting In/Del(s) specific to the cancer cells from cancer cells of a patient; producing a cleavaging agent recognizing In/Del(s); and delivering a composition comprising a nuclease and the cleavaging agent to the patient.

The compositions, methods and uses of the present invention can be utilized in a subject in need thereof in a sufficient amount or in an effective amount. The term "effective amount" or "sufficient amount" is an amount given in one or multiple doses singly or in combination with at least one other therapeutic composition, protocol or therapy regimen that benefit the subject for any period of time or provide the subject with an expected or desired result. The amount prescribed may be varied depending on factors such as the formulation method, mode of administration, age, weight, gender and pathological condition of the patient, administration time, administration route, excretion rate, and response sensitivity.

A vector, such as a viral vector, plasmid vector, or agrobacterium vector, comprising an expression cassette of a cleavaging agent that specifically recognizes a nucleic acid sequence including an insertion and/or deletion specific to cells having genomic sequence variations, for example, cancer cells, and a nuclease or the nucleic acid encoding the same, may be used for delivery. Specifically, as the viral vector, AAV (adeno-associated virus) vector may be used for delivery.

The dosage of AAV vector suitable for achieving a therapeutic effect can be provided as vector genome dose/body weight (vg/kg), and may vary depending on factors such as (a) the route of administration, (b) the expression level of the therapeutic gene required to achieve the therapeutic effect, (c) any host immune response to the AAV vector, and (d) the stability of the expressed protein.

### Example

Hereinafter, the present invention will be described in more detail with reference to examples. However, it will be obvious to those skilled in the art that these examples are provided only for illustration of the present invention and should not be construed as limiting the scope of the present invention.

### Example 1: Detection of cell death effect by DSB

Taking into consideration the fact that plural several DSBs can induce cell death and that all cancer cells have their own In/Del sequences, the unique In/Del sequences of HCT116, U2OS and REP1 cells were identified through WGS (whole-genome sequencing). Then, as shown in Tables 1 to 3 below, among the unique DNA insertion sequences of cancer cells, 30 sequences of regions with an insertion size of 6 to 8 bp evenly distributed on the chromosome were selected to produce crRNA for each cell line, cells were transfected with the CRISPR RNP complex and then crRNA specificity and cell viability were observed.

**[Table 1]**

| SEQ ID NO. | name | Sequence (5'-3') |
|---|---|---|
| 1 | HCT116 1 | AGCCCTAGAATTCCCTTCAC |
| 2 | HCT116 2 | CTTCTCCACCAATTGGTGTT |
| 3 | HCT116 3 | GTTTTGTCTCATTATCACGC |
| 4 | HCT116 4 | CTGTGTTTATGGTGCTTTGT |
| 5 | HCT116 5 | TGTAAGAAGGCCGAATCACG |
| 6 | HCT116 6 | TCCTATACGGCTCTACCAGT |
| 7 | HCT116 7 | GTCTAAAGGTTAGAATTCCG |
| 8 | HCT116 8 | GAGACTGCTATCAGTCATGT |
| 9 | HCT116 9 | TTTTGGTCAAGAGCAGAGGA |
| 10 | HCT116 10 | TGTGTGCCGTAATATGGGAA |
| 11 | HCT116 11 | TGACGTTCTGAGTTCCTTAT |
| 12 | HCT116 12 | GTTTGTCATACCAGTCAAAG |
| 13 | HCT116 13 | ACACAGGACCAGAAACGCTG |
| 14 | HCT116 14 | ACTCTTCCAGTTGTTCACTG |
| 15 | HCT116 15 | GTGCATTTCTCTGCTGAGTC |
| 16 | HCT116 16 | TATATATCTGCAGGATCTGC |
| 17 | HCT116 17 | TCTCTTGCTGTAGAGTGCGT |
| 18 | HCT116 18 | ACACCTGCTTCAGGTGTGTG |
| 19 | HCT116 19 | CATTTAAAAGGATGCCAGCA |
| 20 | HCT116 20 | CTGATACTTCTGATACCAGA |
| 21 | HCT116 21 | GTTCAGCCTGAGTTTGGAGT |
| 22 | HCT116 22 | AGAGATACAGAAGTCCCTGT |
| 23 | HCT116 23 | AGATGTGTAAGGTTGCAACA |
| 24 | HCT116 24 | GAACCACAGAACCTGGCATA |
| 25 | HCT116 25 | TGACCTTTCAC AAAGGCCCA |
| 26 | HCT116 26 | GCCTCAGGGGAATGGAGATA |
| 27 | HGT716 27 | TTTGCTACTTTGCTAGGTTT |
| 28 | HCT116 28 | AGGGAGCTCAGAGTCTTGTG |
| 29 | HCT116 29 | CTCCTTCCCTTCCTGAGTTT |
| 30 | HCT116 30 | GTGTGAGTGAGAGAGAAAGA |

**[Table 2]**

| SEQ ID NO. | name | Sequence (5'-3') |
|---|---|---|
| 31 | U2OS 1 | TTATCCAATCAGCTATGGCC |
| 32 | U2OS 2 | GCTTCACTGGCTTCACTGGA |
| 33 | U2OS 3 | ATTTGTACAGTCTGCTTACT |
| 34 | U2OS 4 | GCATCTTCAACASGTGATTC |
| 35 | U2OS 5 | TTTCAAGCATTTCAATGCAG |
| 36 | U2OS 6 | TTCTCTCTGTGCTTCTTTGA |
| 37 | U2OS 7 | TGGCCCTTGTGGCAGTTTAG |
| 38 | U2OS 8 | ATGGGATTAATGGGATTGCT |
| 39 | U20S 9 | TCATACAGAGAAAGCAGGGC |
| 40 | U2OS 10 | TCATCTCATGTCTTCTCATG |
| 41 | U2OS 11 | GACAGAACCCMGTAATTTC |
| 42 | U2OS 12 | TTATGCCATCTGGTCCAGGC |
| 43 | U2OS 13 | CCAGACATACACTAGGCATC |
| 44 | U2OS 14 | TCCCGTGAGGCATTCTGTAC |
| 45 | U2OS 15 | ATTCTTCGTGCTGATGTACC |
| 46 | U2OS 16 | ACAATCTGTCCAGAGGCCAA |
| 47 | U2OS 17 | GTGAAGGGCAACCAAGGACA |
| 48 | U2OS 18 | TGATATGGCATAGCGATCAT |
| 49 | U2OS 19 | TAACAGCCATGTGGTGTTAC |
| 50 | U2OS 20 | GGAAACAGCAGCAGTGCACA |
| 51 | U2OS 21 | CAGGGCTAGACCTTCGTTAT |
| 52 | U2OS 22 | ATGCAGTGTAGCATGGGGAG |
| 53 | U2OS 23 | AGTCTTTGGACAAGATGCGC |
| 54 | U20S 24 | GAAGAAAGAGAAGAGGGCTT |
| 55 | U2OS 25 | AGCACTTTTATCTCACCCTA |
| 58 | U2OS 26 | ACTGCCTGGGGTTTTCCCTT |
| 57 | U2OS 27 | GTGTCAACAGGGTCACTCTG |
| 58 | U2OS 28 | AATTTGCTTTGGAAGGACCT |
| 59 | U2OS 29 | AGATTCCAGAGTGATGGAAT |
| 60 | U2OS 30 | AGAGATACAGGAGTCCCTGT |

**[Table 3]**

| SEQ ID NO. | name | Sequence (5'-3') |
|---|---|---|
| 61 | RPE1 | GAGTAATAAGTCTGCTCTTT |
| 62 | RPE2 | ACTTTGAGGACCTTGAGGAA |
| 63 | RPE3 | ACTGTGGGAAGTGTGGGAGA |
| 64 | RPE4 | CAGGCATATTTTCCCATGTA |
| 65 | RPE5 | ATGTGATGCTGGAGAGAAAT |
| 66 | RPE6 | GGGGTTAGTTTGTGTTAACT |
| 67 | RPE7 | ACTTACATCACAGGCATCAC |
| 68 | RPE8 | ATGCCAGATTCTTCCCAGTG |
| 69 | RPE9 | ACGAACTGTTGGGTGGTGCT |
| 70 | RPE10 | TTTAATCGAGCACATGCAGG |
| 71 | RPE11 | TGATGGATCTGATGGATACT |
| 72 | RPE12 | AAGAAGGGCTGGTTTGTTCT |
| 73 | RPE13 | ACCTGCAGGAACTGAAACAA |
| 74 | RPE14 | TTGACTCCCATGGTAACCTG |
| 75 | RPE15 | ATGAGGTTACTCAGAGCCAC |
| 76 | RPE16 | CACTTGAGTTCAGGAGAGCT |
| 77 | RPE17 | CTTCACTTCCCTCCTTTCCA |
| 78 | RPE18 | CACTGCCCTCAAGTCCTTAC |
| 79 | RPE19 | CAAACTCACCAAATGTCCAC |
| 80 | RPE20 | TTCTTTGGTTGTGGTGGTTG |
| 81 | RPE21 | TAGTGTTGGGGCATAACACC |
| 82 | RPE22 | GTGGCATTTGGAGTCCATGA |
| 83 | RPE23 | CTCAGTACTTGGTCTCCTGT |
| 84 | RPE24 | ACCTCTTGAGGGGTAACAAA |
| 85 | RPE25 | CCCTGAATACTGAGCAAAGC |
| 86 | RPE26 | GGGCAAGTGTGTGAAGTGTG |
| 87 | RPE27 | ACACAGTAAAGACCCAAGTA |
| 88 | RPE28 | TGTACATTTCCAGGTTCTTC |
| 89 | RPE29 | TTGGCCAGCTTGTCCTGAGT |
| 90 | RPE30 | GGCCAAGATTGCATCCAGTC |

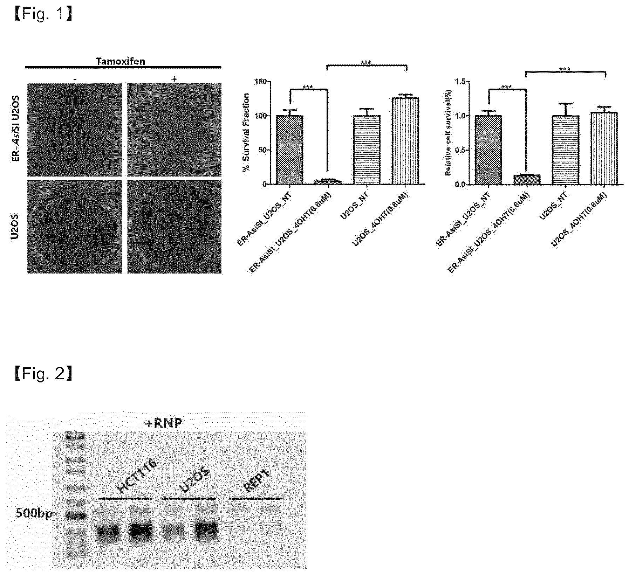

First, an experiment was performed to determine whether or not death of cells are induced when a plurality of DNA DSBs occurs simultaneously. Cell death was detected after treatment of cells obtained by inserting the domain of the estrogen receptor into the AsiSI restriction enzyme with 4-OHT (tamoxifen), and the results are shown in FIG. 1.

When the AsiSI restriction enzyme is treated with 4-OHT, it enters the nucleus and recognizes a specific sequence to create about 100 DNA DSBs (double-strand breaks). The cell viability after production of DSBs was detected through a colony forming assay. Starting 2 days after 200 cells were seeded, the cells were treated with 4-OHT regularly (the culture medium was treated therewith once every 2-3 days) to produce DSBs. After 2 weeks, whether or not colonies formed was determined through methylene blue staining. In addition, since the respective cells have different colony sizes, it was found that destained samples exhibited similar relative cell survival to those of stained samples.

### Example 2. Detection of in-vitro operation of CRISPR system

An *in-vitro* cleavage assay was performed to determine whether or not the CRISPR system operated using the crRNA prepared in Example 1. First, the DNA of the front and rear parts of the insertion sequence was amplified to a size of 500 bp through PCR and then purified, and then whether or not the DNA was cleaved by the CRIPSR system using the prepared crRNA was determined.

Genomic DNA was extracted from RPE1, U2OS and HCT116 cells using the Qiamp DNA mini kit, and bidirectional primers about 500 bp long were specifically produced based on the guide RNA as the center. Genomic DNA was amplified at a set annealing temperature using iProof High-Fidelity DNA polymerase, the amplified DNA was cleaved in 1% agarose gel, and extraction was performed using a Qiagen QiAquick gel extraction kit.

Raw materials were stored at 95°C for 5 minutes and allowed to cool to room temperature to produce a 10 µM crRNA:tracerRNA complex. Then, the crRNA:tracerRNA complex and Cas9 nuclease were each adjusted to a concentration of 1 µM using PBS and incubated for 10 minutes at room temperature to produce an RNP complex.

10X Cas9 Nuclease Reaction Buffer (200 mM HEPES, 1M NaCl, 50 mM MgCl (2), 1 mM EDTA pH 6.5), 1 µM Cas9 RNP, 100 nM DNA substrate, and Nuclease-Free Water were mixed, and a digestion reaction was performed at 37°C for 3 hours. After 3 hours, to release the DNA substrate from Cas9 endonuclease, 1 µl of 20 mg/ml Proteinase K was added and incubated at 56°C for 10 minutes. The cleaved genomic DNA was detected in 1% agarose gel.

**[Table 4]**

| **1. crRNA:tracerRNAcomplex** | Amount (µL) |
|---|---|
| 10 µM Alt-R CRISPR-Cas9 crRNA | 10 |
| 100 µM Alt-R CRISPR-Cas9tracrRNA | 1 |
| Total volume | 100 |

**[Table 5]**

| **2. RNP complex** | Amount (µL) |
|---|---|
| 9uM Complexed crRNA:tracrRNA | 11 |
| Cas9 enzyme [1 µM] | 1.4 |
| PBS | 77.6 |
| Total volume | 90 |

**[Table 6]**

| **3. Digestion** | Amount (µL) |
|---|---|
| 10XCas9 Nuclease Reaction Buffer | 1 |
| **1 uM cas9 RNP** | **2** |
| **100nM DNAsubstrate** | **2** |
| Nuclease-Free Water | 5 |
| Total volume | 10 |

The results are shown in FIG. 2, and two original DNA fragments with a size of 500 bp and a size of 300 bp were detected in an agarose gel. This proved that the CRISPR system using the produced crRNA is desirably worked.

### Example 3. Detection of cell growth rate after DNA DSB induction

Colorectal cancer HCT116 cells and osteosarcoma U2OS cells were transfected with ribonucleotide protein (RNP) complexes with 30 specific sequences to induce DNA DSBs, and then the cell growth rate was detected through a colony forming assay.

500 and 1000 cells were separately seeded and transfected with a cell-line-specific RNP complex. 1000 U2OS cells were seeded on a 60 mm dish and 500 HCT116 cells were seeded on a 60 mm dish, followed by stabilization for two days to prepare for transformation of guide RNA. Raw materials were stored at 95°C for 5 minutes and allowed to cool to room temperature to produce a complex of 1 µM crRNA and 1 µM tracerRNA. Then, 1.5 µl of 1 µM crRNA:tracerRNA complex was reacted with 1.5 µl of 1 µM Cas9 Nuclease and 22 µl of Opti-MEM media on a 96-well dish at room temperature for 5 minutes to produce an RNP complex. 30 RNP complexes (25 µl), 30 x 1.2 µl of RNAiMAX transfection reagent, and 30 x 23.8 µl of Opti-MEM medium were adjusted to 1.5 ml of a total volume and was allowed to stand at room temperature for 20 minutes to produce a transformant complex. In addition, to determine whether or not the induction of DSB using the crRNA specific to one cell line occurred in other cell lines, colorectal cancer cells were transfected with the RNP complex specific to osteosarcoma cells, and the osteosarcoma cells were also transfected with the RNP complex specific to colorectal cancer cells. At this time, 2 µM KU 55933, as an ATM inhibitor, was treated along therewith to inhibit the intracellular DSB repair system. Then, while growth of the cells was monitored, the medium was changed every 3 days and was additionally treated with an ATM inhibitor. After 2 weeks, the growth of the cells was detected through methylene blue staining. The number and area of stained colonies were observed and compared using the ImageJ program, and the results are shown.

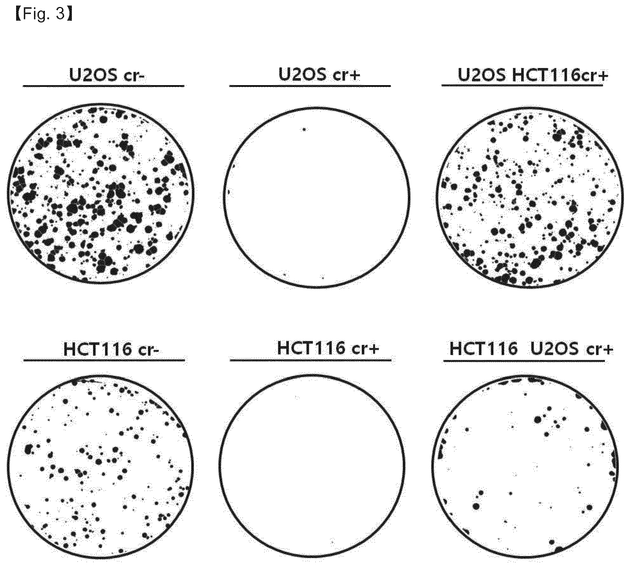

The results are shown in FIG. 3. It was found that in the experimental group transfected with the RNP complex specific to each cell line, the almost of cells were induced to death by DSB, and the specific RNP complex did not induce DSB in other cell lines, so the cells continued to grow.

However, in osteosarcoma transfected with the RNP complex specific to colorectal cancer, cell growth was inhibited to some extent. The reason therefor is considered to be that the growth of cells was inhibited by transfection with a large amount of RNP complexes (30 RNP complexes). In order to compensate for this drawback and determine the minimum RNP complex for cell death, RPE1 cells were transfected with HCT116 and U2OS gRNA to detect the crRNA specificity, and the minimum number of gRNAs is adjusted to 20 per cancer cell.

### Example 4. Detection of effect of CINDELA (Cancer-specific INsertion-DELetions induced Cell death)

A novel U2OS-cell-line-specific crRNA for saCas9 was designed through WGS (whole-genome sequencing) and packaged in AAV. The specific sequences of the U2OS-cell-line-specific crRNA are as follows.

**[Table 7]**

| Numbering | Target Chromosome# | Forward Sequence |
|---|---|---|
| 91 | Chromosome #05 | TACCTATGCCTCATGTAGAAA |
| 92 | Chromosome #13 | ATCGTCAGGTTCTGGGACCGT |
| 93 | Chromosome #03 | CAGAAGAGAGAGAGTAGTAGA |
| 94 | Chromosome #02 | GAATGTTTAAGGTATAGTTTA |
| 95 | Chromosome #03 | TGTTTCAGCAGGGGTTGAAGC |
| 96 | Chromosome #04 | CTATACCCTAGACTTATTCCT |
| 97 | Chromosome #06 | GTTTCTTTCTACAGAATAGAG |
| 98 | Chromosome #01 | ATAGGTTAACAAAGATATTCA |
| 99 | Chromosome #04 | CTACAAAATAGGTGACATAAC |
| 100 | Chromosome #05 | TTAAAATGGCGCAAATAAATT |
| 101 | Chromosome #05 | TTTTATGAGTCTGCCAGGAAT |
| 102 | Chromosome #05 | GAAGAAACAATTTTCACTGGG |
| 103 | Chromosome #01 | CAGGAAGGGAGCTAGTGAGCT |
| 104 | Chromosome #05 | CCTCTCCTGCTGATGATCCCC |
| 105 | Chromosome #01 | TGGGCCGCACGTGTGAGTGCC |
| 106 | Chromosome #04 | AGGAAACAAGCCCATGTTCCC |
| 107 | Chromosome #05 | CATGGCAGAAAACAGAAGACA |
| 108 | Chromosome #05 | AACAGTCTCTGTGATAGGGCA |
| 109 | Chromosome #05 | GGGAAAATCAGACGGATATTC |
| 110 | Chromosome #06 | ATTTCTGTTCTTCCCTCACTT |
| 111 | Chromosome #06 | TAATAACTGTGTAGTTATAAC |
| 112 | Chromosome #05 | GTTCAGATTABCTCTTAACTA |
| 113 | Chromosome #07 | TAAACTTCCTATTTATTGTCT |
| 114 | Chromosome #07 | ATAACTAATGCCAGGGTGAGT |
| 115 | Chromosome #01 | CTCCAGGCCGGTAGGGTTAGA |
| 116 | Chromosome #01 | AGTTTGTAGTGTCTTTCCAGA |
| 117 | Chromosome #04 | TATTTTCCAGATGTTCCAATA |
| 116 | Chromosome #05 | GACTATGAGACTACTACTGCA |
| 119 | Chromosome #06 | CCTTAGCCTCCTTTTCACACA |
| 120 | Chromosome #01 | AGAGAAGAGAAAATAGGGGAA |

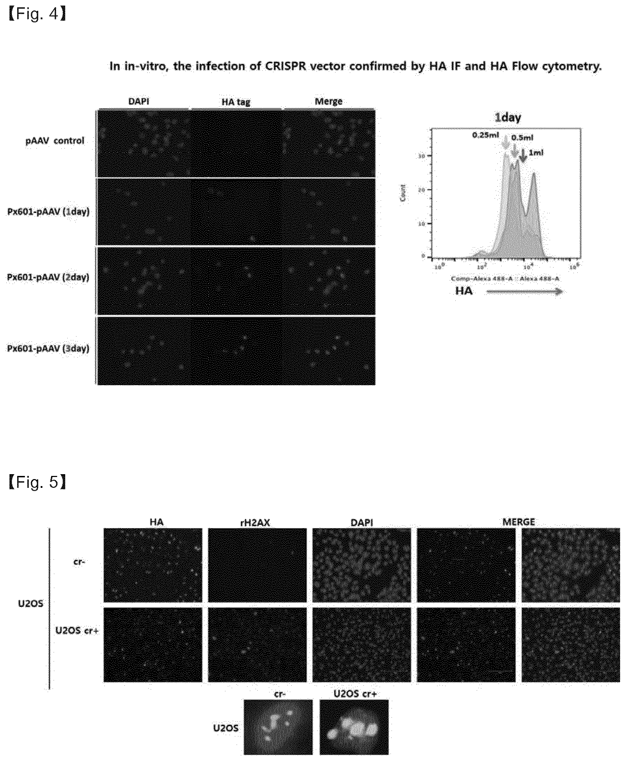

The cell infection rate of AAV was measured using immunofluorescence and flow cytometry. HA intracellular staining was performed by adding 0.1M cells to a 60 mm dish (day 0), not adding HA or adding 0.25 ml, 0.5 ml, 1 ml of HA thereto to transfect the cells with AAV (day 2), and harvesting and then staining the cells (day 3). HA (1:300) was allowed to stand at room temperature for 2 hours, mouse 488 (1:500) was allowed to stand at room temperature for 1 hour, RNAase A was allowed to stand at 37°C for 30 minutes, and PI was allowed to stand at room temperature for 10 minutes. The results are shown in FIG. 4. The percentage of nuclear HA tag-positive cells was about 80% on the 1^{st} day, and the percentage decreased on the 2^{nd} and 3^{rd} days.

8*10⁴ U2OS cells were seeded per well in chamber slides and grown overnight. The cells were washed twice with ice-cold PBS. 1 ml of a cold permeate solution (CSK buffer + 0.5% Triton X-100) was applied thereto for 10 minutes. The cells were washed twice with 1 ml of cold PBS. The cells were fixed at room temperature for 15 minutes using 500 µl of 4% paraformaldehyde in PBS.

The fixing agent was removed and 1 ml of 100% methanol was added thereto, and the cells were then incubated at -20°C for 10 minutes. The cells were washed twice with 1 ml of PBS.

The cells were blocked with 500 µl of 10% FBS in PBS for 30 minutes, the blocking solution was removed, and then an appropriate primary antibody was added to the blocking solution for 1 hour. The cells were washed 3 times with 0.05% Triton X-100 in PBS for 5 minutes. The secondary antibody was added to the cells in the dark at room temperature for 30 minutes, and the cells were washed three times with 0.05% Triton X-100 in PBS for 5 minutes, and were further washed with PBS and DW. The surrounding chamber was removed from the slide.

A mounting reagent was applied dropwise to each sample, and the sample was covered with a cover glass. The sample was dried in that state, and the slide was completely sealed. Focus detection and visualization were performed using an LSM 880 (ZEISS) and ZEN software (ZEISS).

The results are shown in FIG. 5. It was found that AAV particles were sufficiently transfected through immunofluorescence, and the crRNA in Table 7 is capable of generating rH2AX foci corresponding to DNA DSB.

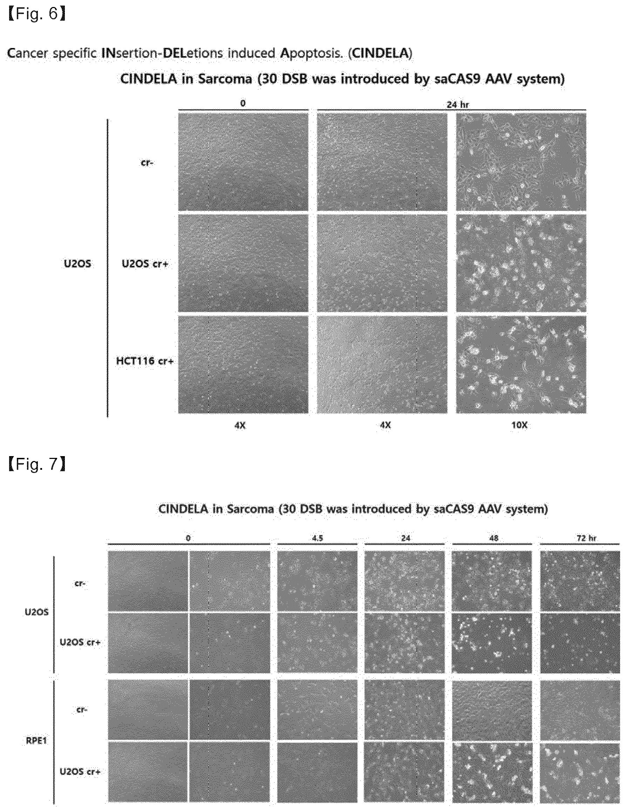

The saCAS9 AAV system was developed using 30 U2OS-cell-line-specific crRNAs, and AAV particles were transduced into U2OS cells. 8 x 10⁴ U2OS cells were plated on a 6-well plate and incubated overnight to transduce the cells with 9 x 10⁹ AAV particles. As can be seen from FIG. 6, U2OS-specific crRNA-dependent cell death was observed through the EVOS cell imaging system after 24 hours. However, non-specific crRNA and HCT116 cell-specific crRNA, were not able to induce cell death. The results of FIG. 6 proved that cell-line-specific selective cell death can be induced by the AAV system.

Whether or not cell-line-specific selective cell death occurred in a normal cell, specifically the RPE1 cell line, was detected. As can be seen from FIG. 7, as in Example 2, the U2OS-cell-line-specific saCAS9 AAV system operates and can induce cell death in U2OS cells, whereas the U2OS-cell-line-specific AAV system did not induce cell death in RPE1 cells.

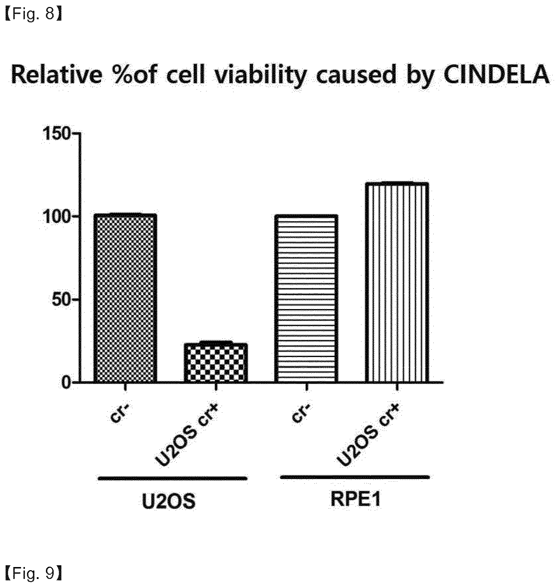

The cell viability due to cancer specific INsertion-DELetions induced Cell death (CINDELA) was measured. After 72 hours, cells transduced with AAV particles were stained with 1% methylene blue at room temperature for 10 minutes. The cells were washed 3 times with PBS for 10 minutes and dried at room temperature. The cells were bleached with 500 µl of a 10% acetic acid solution and the OD value was measured. FIG. 8 shows the resulting cancer cell viability.

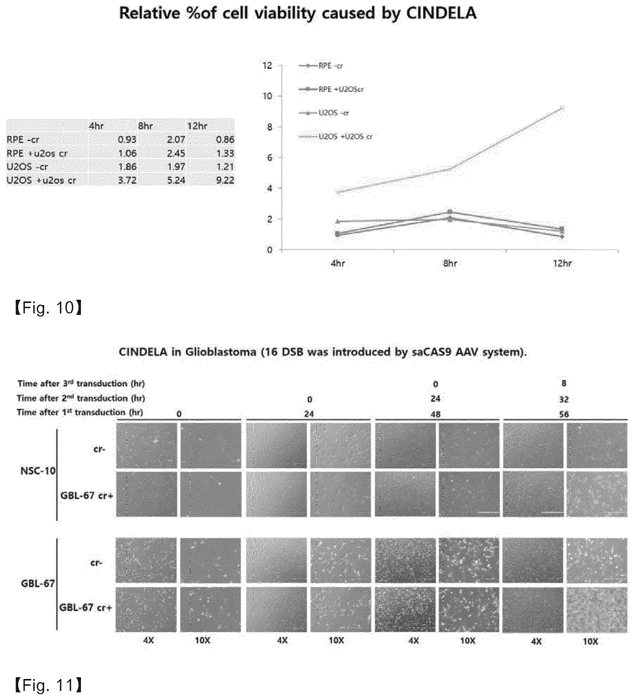

Cell viability was measured in a time-dependent manner through flow cytometry. Both U2OS and RPE1 cells were transduced with U2OS-cell-line-specific-crRNA AAV particles. The cells were resuspended at 1*10⁶/ml using abd serum not containing azide/PBS not containing protein. 1 µl of pf FVD was added with respect to 1 ml of the cell, followed by vortexing immediately. The result was incubated at 4°C for 30 minutes and light was blocked. The cells were washed twice with PBS, and the FACS VERSE (BD Inc.) machine was checked. As can be seen from FIG. 9, AAV-dependent cell death was induced only in crRNA-specific cell lines.

### Example 5. Detection of effect of glioblastoma-specific INDEL induced cell death

The cell death effect due to IN/DEL was detected in patient-derived glioblastoma. The experiment was performed in the same manner as in Example 4, except that the cells were incubated in a hypoxic chamber. There were only 16 sequences available for glioblastoma. The detection of selective cell death through 16 glioblastoma-specific sequences means production of only 16 DNA DSBs.

**[Table 8]**

| Numbering | Target Chromosome# | Forward Sequence |
|---|---|---|
| 121 | Chromosome #05 | ACACGGACAAGTTCTCCGTGA |
| 122 | Chromosome #13 | AACAGTGGGGCAGTTAGGATT |
| 123 | Chromosome #03 | CACAAGATAGATAGA7AAGAT |
| 124 | Chromosome #02 | TTTCCAGGTTTTGATTGAGGT |
| 125 | Chromosome #03 | CAGATGCCAGAAAGGAGACTG |
| 126 | Chromosome #04 | GAGGAGAAGCGGCGATAATCT |
| 127 | Chromosome #06 | GTAGAAGGCTAAATAGTTACC |
| 128 | Chromosome #01 | TTATAACTAAGATTCTGGCCC |
| 129 | Chromosome #04 | TCTCTTTGCACCCCAGGCATG |
| 130 | Chromosome #05 | AGATAACAATAATTATTACTT |
| 131 | Chromosome #05 | CCCCCTGGCCASGGAGGGCCG |
| 132 | Chromosome #05 | AGTAGCACGAACAAAACAAGT |
| 133 | Chromosome #01 | GAGAAAAATCAGGATAGAGGT |
| 134 | Chromosome #05 | CTCCACAAGCAGATGATCA AG |
| 135 | Chromosome #01 | AAGTTTTGTAGAAAACTAAAT |
| 136 | Chromosome #05 | TACTGTGGGATAACTGACGGC |

Serial transduction was performed for 16 glioblastoma specific sequences. NSC-10 cells were used as normal controls. As can be seen from FIG. 10, selective cancer cell death occurred in glioblastoma.

### Example 6. Effect of cell death depending on presence of ATM kinase

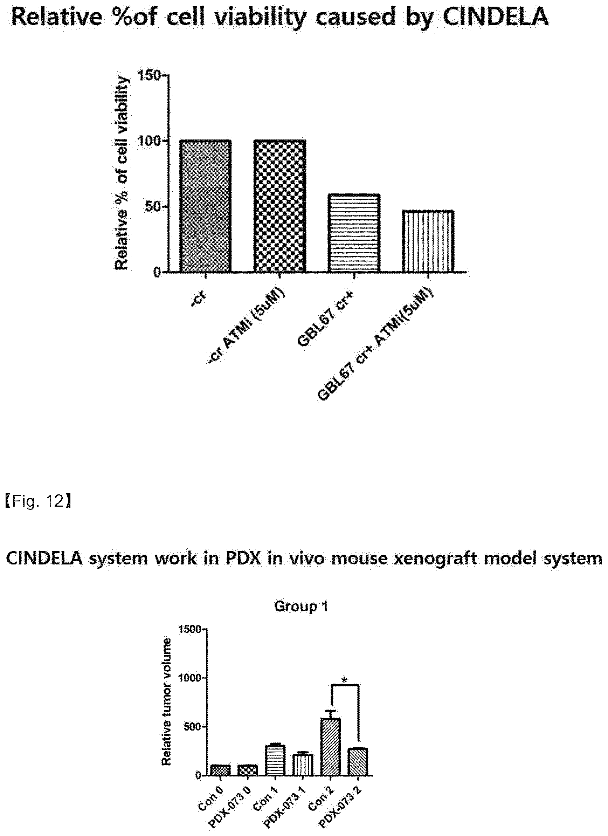

AAV particles containing or not containing an ATM kinase inhibitor were transduced into the glioblastoma cells of Example 5, and an experiment was conducted in the same manner as in Examples 4 and 5. After 24 hours of transduction, the cells were stained with 1% methylene blue at room temperature for 10 minutes. The cells were washed 3 times with PBS for 10 minutes and dried at room temperature. The cells were bleached with 500 ul of 10% acetic acid solution and OD was measured. The results are shown in FIG. 11.

### Example 7. Detection of effect of lung cancer-specific IN/DEL-induced cell death

In order to detect the effect of cancer-specific IN/DEL-induced cell death (CINDELA), lung cancer tissue derived from a patient was used for mouse xenograft. The patient-derived lung cancer tissue was inserted into the mouse, and AAV particles containing 28 lung cancer tissue-specific crRNA were continuously injected into the mouse.

**[Table 9]**

| numbering | Target Chromosome# | Forward Sequence |
|---|---|---|
| 137 | 1 | GAGGCTATTGGATTTCATTTCTAGAGT |
| 138 | 1 | GCACTCACGAGGTCACGAGGTGTGGGT |
| 139 | 2 | CTTTCTTAAACATAGAATCTATAGGAT |
| 140 | 2 | GAACAGTGCAAGGATA3GTGTGGGGGT |
| 141 | 3 | TGGTGCCCCGGGTTTACACTTAAGAAT |
| 142 | 3 | CTTCATCTATAGGAGCCTCCAGTGAGT |
| 143 | 4 | GGCCTTGAGTGAGGAGAAGGCAGGAGT |
| 144 | 5 | GGTGAAGTACATATTCTCATATGGAGT |
| 145 | 5 | GGGCTCAGTTTTCCCACCAGTGGGGGT |
| 146 | 6 | ATACGTTTTGACggCCAATAGTTGAAT |
| 147 | 8 | ACCTATGATGTGATAGTTTGTTTGGGT |
| 148 | 8 | TAAGACCTCTTAGGAAGTAGAATGAAT |
| 149 | 9 | TTTGAGAGGCAGGGGCACCAGCTGGGT |
| 150 | 9 | TGGAAGAGTGGAAAAAGGTGGAAGAGT |
| 151 | 10 | TCTGCAGAACAGGCOCCCAGTCAGAGT |
| 152 | 10 | TGTAGAATTTTTAACTGTTAACAGGAT |
| 157 | 12 | GCATCACTGAAGAATCAGCCTAAGAGT |
| 158 | 13 | GATTTTAATCATAACTGCATGAAGGGT |
| 159 | 14 | GTCACAAGTTTCTGTTTCTTGGTGGAT |
| 160 | 15 | TCCATCTCTGAAATGTGGATGGAGAAT |
| 161 | 16 | TAAAGGGTGCTTTTCTTATTATAGAAT |
| 162 | 18 | TTTGGGAGTGGAGAGATTTGGGGGAGT |
| 163 | 20 | AGGCTCTCAGGGAATGAGAGGAGGGAT |

AAV particles were injected every 2 days, and tumor size was measured. The results are shown in FIG. 12. As can be seen from FIG. 12, normal cells grew over time, whereas cancer cells did not grow during the second AAV injection.

Although specific configurations of the present invention have been described in detail, those skilled in the art will appreciate that this description is provided to set forth preferred embodiments for illustrative purposes and should not be construed as limiting the scope of the present invention. Therefore, the substantial scope of the present invention is defined by the accompanying claims and equivalents thereto.

### [Sequence Free Text]

An electronic file is attached.

## Claims

1. A composition for inducing death of cells having genomic sequence variations comprising a nuclease and a cleavaging agent that specifically recognizes a nucleic acid sequence comprising a mutant sequence specific to cells having genomic sequence variations.

2. A composition for treating a cancer comprising a nuclease and a cleavaging agent that specifically recognizes a nucleic acid sequence comprising an insertion and/or deletion specific to the cancer.

3. The composition according to claim 1 or 2, wherein the nuclease is a restriction enzyme, a zinc finger nuclease (ZNFN), a transcriptional activator-like effector nuclease (TALEN), or a Cas protein.

4. The composition according to claim 3, wherein the Cas protein is Cas3, Cas9, Cpf1, Cas6, C2c12, or C2c2.

5. The composition according to claim 3, wherein the Cas protein is derived from a microorganism genus comprising an ortholog of a Cas protein selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus (Staphylococcus aureus), Nitratifractor, Corynebacterium* and *Campylobacter,* and wherein the Cas protein is isolated therefrom or recombined.

6. The composition according to claim 1 or 2, wherein the cleavaging agent is guide RNA comprising at least one sequence selected from the group consisting of sequences represented by SEQ ID NOS: 1 to 163.

7. The composition according to claim 1, wherein cells having genomic sequence variations are cancer cells.

8. The composition according to claim 1 or 2, wherein the cleavaging agent that specifically recognizes the nucleic acid sequence including an insertion and/or deletion specific to normal cells is guide RNA including at least one sequence selected from the group consisting of sequences represented by SEQ ID NOS: 61 to 90.

9. The composition according to claim 2, wherein the cancer is colorectal cancer, and the cleavaging agent that specifically recognizes the nucleic acid sequence comprising an insertion and/or deletion specific to the colorectal cancer is guide RNA comprising at least one sequence selected from the group consisting of sequences represented by SEQ ID NOS: 1 to 30.

10. The composition according to claim 2, wherein the cancer is osteosarcoma, and the cleavaging agent that specifically recognizes the nucleic acid sequence comprising an insertion and/or deletion specific to the osteosarcoma is guide RNA comprising at least one sequence selected from the group consisting of sequences represented by SEQ ID NOS: 31 to 60.

11. The composition according to claim 2, wherein the cancer is glioblastoma, and the cleavaging agent that specifically recognizes the nucleic acid sequence comprising an insertion and/or deletion specific to the glioblastoma is guide RNA comprising at least one sequence selected from the group consisting of sequences represented by SEQ ID NOS: 121 to 136.

12. The composition according to claim 2, wherein the cancer is lung cancer, and the cleavaging agent that specifically recognizes the nucleic acid sequence comprising an insertion and/or deletion specific to the lung cancer is guide RNA comprising at least one sequence selected from the group consisting of sequences represented by SEQ ID NOS: 137 to 163.

13. The composition according to claim 1 or 2, wherein the composition induces death of cells by inducing a double-stranded break (DSB) in a region of the nucleic acid sequence comprising the insertion and/or deletion specific to cells having genomic sequence variations.

14. The composition according to claim 1 or 2, wherein the cleavaging agent is introduced using a vector.

15. The composition according to claim 14, wherein the vector is AAV.

16. A method for inducing death of cells having genomic sequence variations, comprising treating cells having genomic sequence variations with a nuclease and a cleavaging agent that specifically recognizes a nucleic acid sequence comprising a mutant sequence specific to cells having genomic sequence variations.

17. A method for inducing death of cells having genomic sequence variations comprising:
performing whole-genome sequencing (WGS) on cells having genomic sequence variations and normal cells;
comparing the resulting WGS data between cells having genomic sequence variations and the normal cells to select a mutant sequence specific to cells having genomic sequence variations;
producing a cleavaging agent that recognizes the selected mutant sequence;
preparing a composition comprising a cleavaging agent and a nuclease; and
applying the composition to cells having genomic sequence variations.

18. A method for inducing death of cells having genomic sequence variations comprising:
performing whole-genome sequencing (WGS) on cells having genomic sequence variations and normal cells;
comparing the resulting WGS data between cells having genomic sequence variations and the normal cells to select In/Del(s) specific to cells having genomic sequence variations;
producing a cleavaging agent that recognizes selected In/Dels;
preparing a composition comprising a cleavaging agent and a nuclease; and
applying the composition comprising the nuclease and the cleavaging agent to cells having genomic sequence variations.

19. The method according to claim 17 or 18, wherein the nuclease is a restriction enzyme, a zinc finger nuclease (ZNFN), a transcriptional activator-like effector nuclease (TALEN), or a Cas protein.

20. The method according to claim 19, wherein the Cas protein is Cas3, Cas9, Cpf1, Cas6, or C2c2.

21. The method according to claim 19, wherein the Cas protein is derived from a microorganism genus comprising an ortholog of a Cas protein selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus (Staphylococcus aureus), Nitratifractor, Corynebacterium* and *Campylobacter,* and wherein the Cas protein is isolated therefrom or recombined.

22. The method according to claim 17 or 18, wherein the cleavaging agent is guide RNA comprising at least one sequence selected from the group consisting of sequences represented by SEQ ID NOS: 1 to 163.

23. The method according to claim 17 or 18, wherein cells having genomic sequence variations are cancer cells.

24. The method according to claim 17 or 18, wherein the cleavaging agent that specifically recognizes the nucleic acid sequence comprising an insertion and/or deletion specific to normal cells is guide RNA comprising at least one sequence selected from the group consisting of sequences represented by SEQ ID NOS: 61 to 90.

25. The method according to claim 23, wherein the cancer is colorectal cancer, and the cleavaging agent that specifically recognizes the nucleic acid sequence comprising an insertion and/or deletion specific to the colorectal cancer is guide RNA comprising at least one sequence selected from the group consisting of sequences represented by SEQ ID NOS: 1 to 30.

26. The method according to claim 23, wherein the cancer is osteosarcoma, and the cleavaging agent that specifically recognizes the nucleic acid sequence comprising an insertion and/or deletion specific to the osteosarcoma is guide RNA comprising at least one sequence selected from the group consisting of sequences represented by SEQ ID NOS: 31 to 60.

27. The method according to claim 23, wherein the cancer is glioblastoma, and the cleavaging agent that specifically recognizes the nucleic acid sequence comprising an insertion and/or deletion specific to the glioblastoma is guide RNA comprising at least one sequence selected from the group consisting of sequences represented by SEQ ID NOS: 121 to 136.

28. The method according to claim 23, wherein the cancer is lung cancer, and the cleavaging agent that specifically recognizes the nucleic acid sequence comprising an insertion and/or deletion specific to the lung cancer is guide RNA comprising at least one sequence selected from the group consisting of sequences represented by SEQ ID NOS: 137 to 163.

29. The method according to claim 17 or 18, wherein the composition induces death of cells by simultaneously inducing a plurality of double-stranded breaks (DSBs) in a region of the nucleic acid sequence comprising the insertion and/or deletion specific to cells having genomic sequence variations.

30. The method according to claim 17 or 18, further comprising treating the cells with at least one ATM (Ataxia telangiectasia mutated) inhibitor selected from the group consisting of caffeine, wortmannin, CP-466722, KU-55933, KU-60019 and KU-559403, at least one ATR (Ataxia telangiectasia and Rad-3 mutated) inhibitor selected from the group consisting of Schisandrin B, NU6027, NVP-BEZ235, VE-821, VE-822 (VX-970), AZ20 and AZD6738, or a DNA double-strand repair inhibitor of DNA-PKcs (DNA-dependent protein kinase catalytic subunit).

31. A method of treating cancer comprising administering a nuclease and a cleavaging agent that specifically recognizes a nucleic acid sequence comprising a mutant sequence specific to cells having genomic sequence variations and a nuclease to a subject.

32. A method of treating cancer comprising treating cells having genomic sequence variations with a vector comprising an expression cassette of a cleavaging agent that specifically recognizes a nucleic acid sequence comprising an insertion and/or deletion specific to cells having genomic sequence variations and a nuclease.

33. The method according to claim 32, wherein the vector is adeno-associated virus (AAV).

34. A composition for patient-specific cancer therapy comprising a nuclease and a cleavaging agent that specifically recognizes a nucleic acid sequence comprising an insertion and/or deletion specific to cancer cells of the patient.

35. The composition according to claim 34, wherein the nuclease is a restriction enzyme, a zinc finger nuclease (ZNFN), a transcriptional activator-like effector nuclease (TALEN), or a Cas protein.

36. The composition according to claim 35, wherein the Cas protein is Cas3, Cas9, Cpf1, Cas6, C2c12, or C2c2.

37. The composition according to claim 36, wherein the Cas protein is derived from a microorganism genus comprising an ortholog of a Cas protein selected from the group consisting of *Corynebacter, Sutterella, Legionella, Treponema, Filifactor, Eubacterium, Streptococcus (Streptococcus pyogenes), Lactobacillus, Mycoplasma, Bacteroides, Flaviivola, Flavobacterium, Azospirillum, Gluconacetobacter, Neisseria, Roseburia, Parvibaculum, Staphylococcus (Staphylococcus aureus), Nitratifractor, Corynebacterium* and *Campylobacter,* and wherein the Cas protein is isolated therefrom or recombined.

38. The composition according to claim 34, wherein the cleavaging agent is guide RNA comprising at least one sequence selected from the group consisting of sequences represented by SEQ ID NOS: 1 to 163.

39. The composition according to claim 34, wherein a cleavaging agent that specifically recognizes a nucleic acid sequence comprising an insertion and/or deletion specific to normal cells is guide RNA comprising at least one sequence selected from the group consisting of sequences represented by SEQ ID NOS: 61 to 90.

40. The composition according to claim 34, wherein the cancer is colorectal cancer, and a cleavaging agent that specifically recognizes a nucleic acid sequence comprising an insertion and/or deletion specific to the colorectal cancer is guide RNA comprising at least one sequence selected from the group consisting of sequences represented by SEQ ID NOS: 1 to 30.

41. The composition according to claim 34, wherein the cancer is osteosarcoma, and a cleavaging agent that specifically recognizes a nucleic acid sequence comprising an insertion and/or deletion specific to the osteosarcoma is guide RNA comprising at least one sequence selected from the group consisting of sequences represented by SEQ ID NOS: 31 to 60.

42. The composition according to claim 34, wherein the cancer is glioblastoma, and a cleavaging agent that specifically recognizes a nucleic acid sequence comprising an insertion and/or deletion specific to the glioblastoma is guide RNA comprising at least one sequence selected from the group consisting of sequences represented by SEQ ID NOS: 121 to 136.

43. The composition according to claim 34, wherein the cancer is lung cancer, and a cleavaging agent that specifically recognizes a nucleic acid sequence comprising an insertion and/or deletion specific to the lung cancer is guide RNA comprising at least one sequence selected from the group consisting of sequences represented by SEQ ID NOS: 137 to 163.

44. The composition according to claim 34, wherein the composition induces death of cells by inducing a double-stranded break (DSB) in a region of the nucleic acid sequence comprising the insertion and/or deletion specific to cells having genomic sequence variations.

45. The composition according to claim 34, wherein the cleavaging agent is introduced using a vector.

46. The composition according to claim 45, wherein the vector is AAV.

47. A patient-specific cancer therapy comprising:
selecting In/Del(s) specific to the cancer cells from cancer cells of a patient;
producing a cleavaging agent recognizing In/Del(s); and
delivering a composition comprising a nuclease and the cleavaging agent to the patient.
